# FASCICULE DE BREVET EUROPEEN

(11) **EP 1 948 710 B1**
(45) Date de publication et mention de la délivrance du brevet: **15.09.2010**
(21) Numéro de dépôt: 06830004.5
(22) Date de dépôt: 15.11.2006
(51) Int. Cl.: C08G 63/82, A61K 9/20

(54) **PROCEDE DE PREPARATION DE POLYHYDROXYALKANOATES, POLYMERES AINSI OBTENUS, COMPOSITIONS LES COMPRENANT, ET LEURS UTILISATIONS**
VERFAHREN ZUR HERSTELLUNG VON POLYHYDROXYALKANOATEN, SO ERHALTENE POLYMERE, ZUSAMMENSETZUNGEN DAMIT UND VERWENDUNG DAVON
PROCESS FOR PREPARING POLYHYDROXYALKANOATES, POLYMERS THUS OBTAINED, COMPOSITIONS COMPRISING SAME AND USE THEREOF

(30) Priorité: 15.11.2005 FR 0553471
(43) Date de publication de la demande: 30.07.2008
(73) Titulaire: ARKEMA FRANCE, 92700 Colombes (FR); Centre National de la Recherche Scientifique, 75016 Paris (FR)
(72) Inventeur: CARPENTIER, Jean-François, F-35690 Acigne (FR); THOMAS, Christophe, F-22690 La Vicomte Sur Rance (FR); AMGOUNE, Abderramane, F-35700 Rennes (FR)
(74) Mandataire: Albani, Dalila
(86) Numéro de dépôt international: PCT/EP2006/068522
(87) Numéro de publication internationale: WO 2007/057422

(56) Documents cités:
- US-A- 5 440 007
- KRICHELDORF ET AL.: "Polylactones. 28. Syndiotactic Poly(B-D,L-hydroxybutyrate) by ring-opening polymerization of B-D,L-Butyrolactone with butyltin methoxides" MACROMOLECULES, vol. 27, 1994, pages 3139-3146, XP002388835 cité dans la demande
- KRICHELDORF AND EGGERSSTEDT: "Polylactones. 41. Polymerizations of B-D,L-butyrolactone with dialkyltinoxides as initiators" MACROMOLECULES, vol. 30, 1997, pages 5693-5697, XP002388836
- KEMNITZER ET AL.: "Preparation of predominantly syndiotactic poly(B-hydroxybutyrate) by the tributyltin methoxide catalyzed ring-opening polymerization of racemic B-butyrolactone" MACROMOLECULES, vol. 26, 1993, pages 1221-1229, XP002388837 cité dans la demande
- AMGOUNE A. ET AL.: "Ring opening polymerization of lactide with group 3 metal complexes supported by dianionic alkoxy-amino-bisphenolate ligands: combining high activity, productivity, and selectivity" CHEM. EUR. J., vol. 12, 14 octobre 2005 (2005-10-14), pages 169-179, XP002388838

## Description

La présente invention a trait à un procédé de préparation de polyhydroxyalkanoates (PHAs), en particulier de poly(3-hydroxybutyrate) (PHB).

Plus précisément, l'invention concerne un procédé de préparation de polyhydroxyalkanoates, tels que le poly(3-hydroxybutyrate) (PHB) par polymérisation par ouverture de cycle de lactones, telles que la β-butyrolactone (BBL).

L'invention concerne également les polymères de PHA susceptibles d'être obtenus par ce procédé, les compositions comprenant ces polymères, et les utilisations de ces polymères et compositions.

Le domaine technique de l'invention peut être défini de manière très large comme celui des polymères biodégradables et de leur préparation.

Les polymères biodégradables ont récemment fait l'objet d'un grand intérêt pour remplacer les matériaux synthétiques classiques [1]. Parmi les nouveaux polymères biodégradables qui ont été développés durant les dix dernières années, les polyhydroxyalkanoates (PHAs) sont particulièrement intéressants. Les propriétés des PHAs vont de rigides à élastiques, en fonction de la longueur des chaînes latérales ou du type de copolymère.

De plus, ces matériaux combinent les propriétés de film barrière des polyesters avec les bonnes propriétés mécaniques du polyéthylène et du polypropylène préparés à partir du pétrole.

Le PHA le plus courant est le poly(3-hydroxybutyrate) (PHB) qui est un polyester aliphatique produit par des bactéries et d'autres organismes vivants [2], [3]. Ce polymère naturel biodégradable et biocompatible est isotactique avec tous les centres stériques dans la configuration (R) [4], [5].

Dans le document [6], on utilise des bactéries qui fabriquent naturellement des polymères et on transforme leur métabolisme pour qu'elles produisent des polymères et copolymères de PHB biodégradables, à partir de sucres végétaux ou d'huiles végétales.

Le PHB produit par des bactéries, isolé, est hautement cristallin, se trouve dans la forme énantiomériquement pure où tous les centres stériques sont dans la configuration (R), fond à 175-180°C, avec une température de transition vitreuse (Tg) de 9°C [7].

Cependant, le fort caractère cristallin et la faible stabilité thermique du PHB sont la source de difficultés lors de son traitement à l'état fondu, car sa température de dégradation (qui donne des groupes crotonates) commence immédiatement au-dessus de la température de fusion (Tₘ), ce qui limite leur importance industrielle.

Pour améliorer l'aptitude au traitement, un PHB syndiotactique, contenant en alternance des séquences formées de motifs compétitifs de (R) et (S), β-hydroxybutyrate pourrait être une alternative intéressante en tant que matière plastique industrielle biodégradable, à la condition que sa température de fusion soit inférieure à celle du polyester isotactique.

De nombreux procédés existent pour la synthèse des PHAs, tels que le PHB, mais le plus commode et le plus prometteur est la polymérisation par ouverture de cycle ("Ring-Opening Polymerization" ou "ROP", en anglais) de lactones, telles que la β-butyrolactone, où la force motrice de la polymérisation est la relaxation de la contrainte du cycle [8].

Il a été montré qu'un polyhydroxybutyrate fortement isostatique (R ou S) pouvait être obtenu lorsque de la β-butyrolactone, optiquement pure (R), respectivement (S), était mise en oeuvre [9], alors que lorsqu'on utilise un mélange racémique de β-butyrolactone (R) et (S), du PHB atactique [10] et du PHB enrichi en diades isotactique [11] ou syndiotactique [12] peut être formé.

Des microstructures isotactiques, atactique ou syndiotactique ont pu être observées lors de la polymérisation, à partir de BBL racémique en utilisant des initiateurs métalliques.

Cependant, à l'exception des catalyseurs distannoxane [12a] et alkylzinc alcoolate [10c], récemment mentionnés, la plupart des systèmes étudiés par la polymérisation par ouverture de cycle de la BBL sont extrêmement lents et/ou ne sont pas capables de produire un PHB de masse moléculaire élevée de manière contrôlée. Dans un document récent, COATES et Al. [13], ont décrit que les complexes de β-diiminate de zinc alcoolates sont capables de polymériser la BBL avec des activités jamais atteintes jusqu'alors, dans des conditions douces, pour préparer des PHA de manière contrôlée. Des masses moléculaires élevées ont été atteintes à température ambiante avec une polydispersité tout à fait bonne (PDI = 1,20) après 12 h, mais tous les PHB obtenus étaient atactiques.

Récemment ont été divulgués plusieurs complexes à site unique bis (phénolate) de métaux du groupe 3, bien définis, qui ont une action efficace en tant qu'initiateurs dans la synthèse de polymères biodégradables, par exemple dans la préparation de poly(acide lactique) hétérotactique et syndiotactique à partir de lactide racémique et de lactide-méso, respectivement [14].

Les PHAS produits par synthèse bactérienne restant, malgré de nombreuses utilisations potentielles, trop chers pour une large utilisation, il existe, au regard de ce qui précède, un besoin pour un procédé de préparation par synthèse chimique qui permette de préparer des PHAs tels que la PHB, notamment syndiotactiques avec un polydispersité étroite, une masse moléculaire élevée, de manière rapide, sélective, et avec un haut rendement.

Le but de la présente invention et de fournir un procédé de préparation de polyhydroxyalcanoates PHA et en particulier de PHB qui réponde, entre autres, à ces besoins.

Le but de la présente invention est encore de fournir un tel procédé de préparation qui ne présente pas les inconvénients, défauts, limitations et désavantages des procédés de l'art antérieur et qui résolve les problèmes des procédés de l'art antérieur.

Ce but et d'autres encore, sont atteints, conformément à l'invention par un procédé de préparation d'un polymère de polyhydroxyalcanoate (PHA) par polymérisation par ouverture de cycle d'une lactone de formule (I) 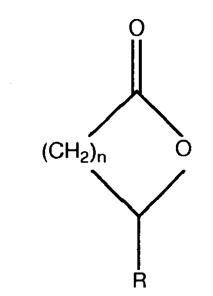 où n est un nombre entier de 1 à 4, et R représente un hydrogène ou un groupe alkyle de 1 à 4C linéaire ou ramifié, caractérisé en ce que la polymérisation est effectuée en présence d'un initiateur de formule (II): 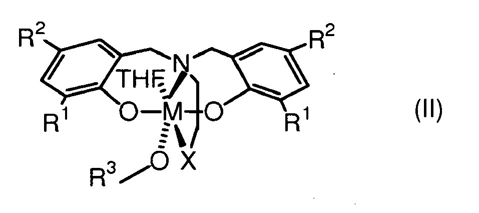 dans laquelle :
- R³ représente un groupe alkyle de 1 à 15C tel qu'un groupe méthyle, éthyle, i-propyle, n-propyle, n-butyle, i-butyle, t-butyle ; un groupe benzyle ;
- R₁ et R₂ identiques ou différents représentent chacun un groupe choisi parmi les groupes alkyle de 1 à 15C, tels qu'un méthyle ; t-butyle ; cumyle (α,α-diméthylbenzyle) ; adamantyle ; trityle ; et trialkyl silyle (C₃ à C₁₅) ;
- X représente O(R₄), S(R₄) ou N(R₄)(R₅), où R₄ et R₅ représentent chacun indépendamment un groupe alkyle de 1 à 15C tel qu'un groupe méthyle, éthyle ; un groupe benzyle ;
- M est un métal du groupe 3 de la classification périodique des éléments tel que Y, La ou Nd.

La lactone est choisie généralement parmi les lactones répondant aux formules suivantes : 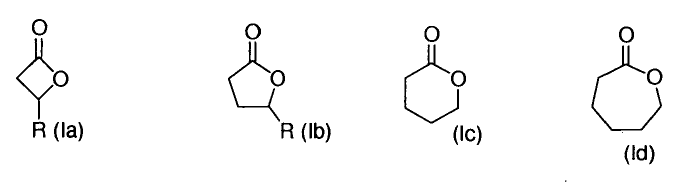 dans lesquelles R a la signification déjà donnée plus haut. De préférence, dans la formule (Ia) R représente H, méthyle ou éthyle et de préférence dans la formule (Ib) R représente H, méthyle, éthyle ou propyle.

La lactone dont on effectue préférentiellement la polymérisation est la β-butyrolactone notamment la β-butyrolactone racémique.

La lactone que l'on polymérise est de préférence une lactone racémique lorsque sa structure le permet c'est-à-dire lorsque dans les formules II ci-dessus R est différent de H. La lactone pourra ainsi être la β-butyrolactone racémique ou la γ-valérolactone racémique. Le procédé selon l'invention permet alors d'obtenir de manière très préférentielle le polymère de PHA (par exemple de PHB) syndiotactique avec un degré de syndioacticité généralement de 70 à 99%.

La Tm de tels polymères varie généralement de 130 à 180°C selon la syndiotacticité

L'initiateur de formule (II) est choisi généralement parmi les composés de formules suivantes :

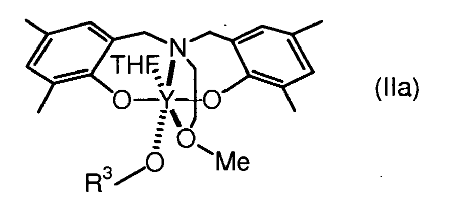

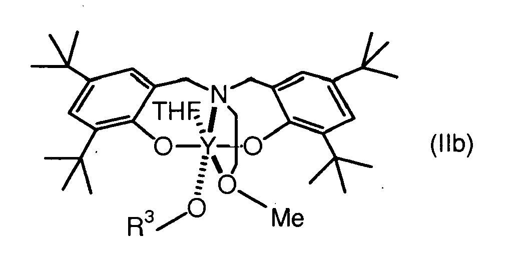

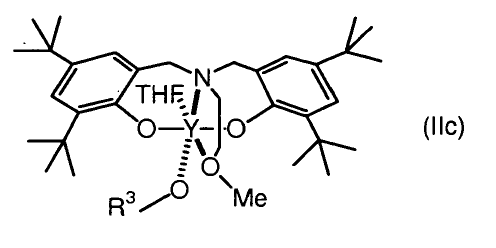

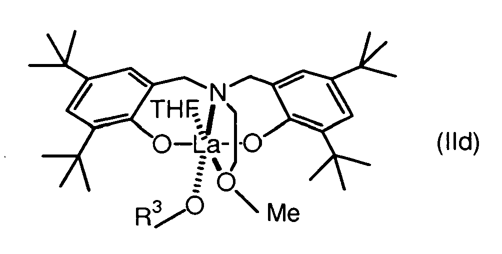

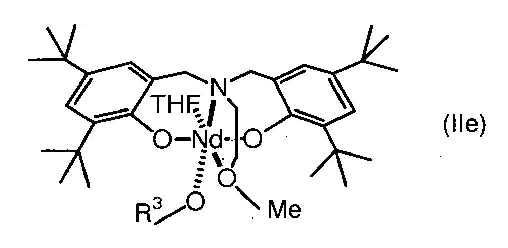

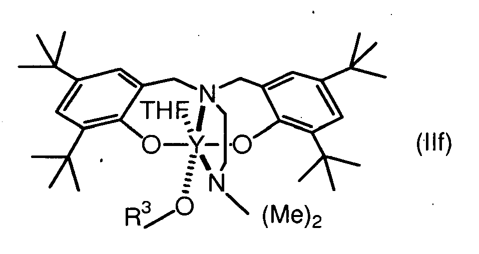

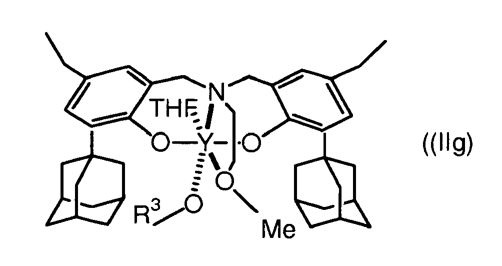

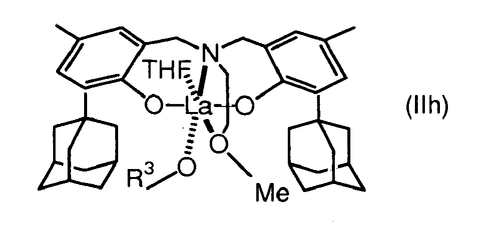

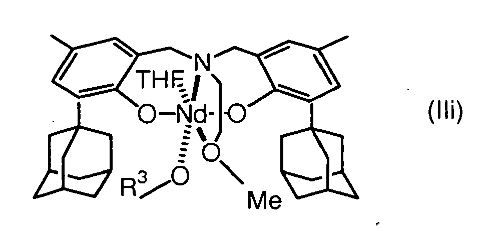

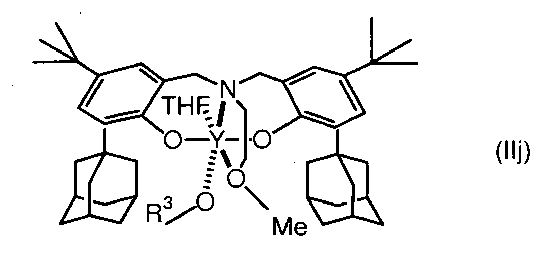

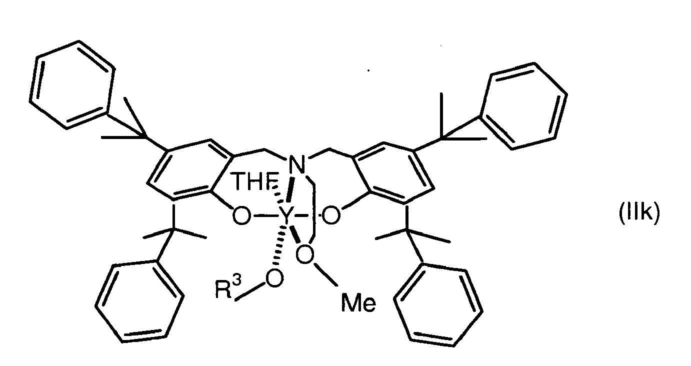

L'initiateur de formule (II) préféré est le composé suivant :

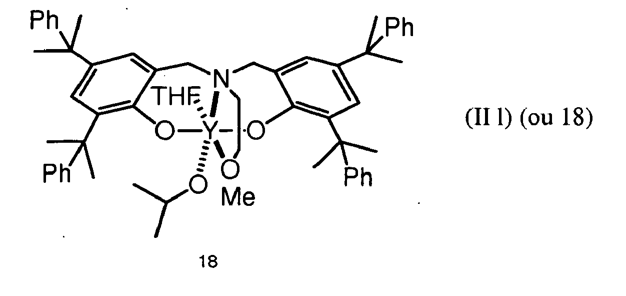

La polymérisation est généralement réalisée dans un solvant choisi parmi, par exemple, le toluène, le tétrahydrofurane (THF), le chlorobenzène et leurs mélanges.

Le solvant préféré est le toluène.

Le rapport de la concentration en lactone à celle de l'initiateur est généralement de 50 à 5000, de préférence de 100 à 2000.

La polymérisation est généralement réalisée à une température de -30 à 120°C, de préférence de 0 à 60°C, et de préférence encore de 15 à 30°C, par exemple de 20°C.

Le procédé selon l'invention s'applique en particulier à la polymérisation de la β-butyrolactone racémique, réalisée avec le complexe de formule (III), dans le toluène, à une température de -30°C à 110°C, de préférence de 0 à 60°C, et le rapport de la concentration en β-butyrolactone sur celle de l'initiateur (du complexe) est généralement de 100 à 2000.

Le polymère préparé a généralement une masse moléculaire moyenne en nombre de 4000 à 500 000, de préférence de 8000 ou 9000 à 170 000.

En outre, le polymère a généralement un indice de polydispersité (PDI) de 1,01 à 1,50, de préférence de 1,05 à 1,20.

Le procédé selon l'invention se distingue fondamentalement des procédés de l'art antérieur car il met en oeuvre pour la polymérisation de lactones par ouverture de cycle des composés initiateurs spécifiques qui n'ont jamais été utilisés dans la polymérisation de lactones par ouverture de cycles, et qu'il conduit à des PHA à haute teneur syndiotactique.

Certains de ces initiateurs ont déjà été utilisés pour la polymérisation par ouverture de cycle de lactides, mais rien ne pouvait laisser supposer qu'ils pourraient être également employés avec succès à la polymérisation des lactones. En effet, les initiateurs sont généralement spécifiques à la polymérisation par ouverture de cycle d'une classe de composés spécifiques et les excellents résultats obtenus avec certains initiateurs pour la polymérisation des lactides ne pouvaient en aucun cas laisser présager que de tels initiateurs seraient aussi avantageux dans le cadre la polymérisation des lactones.

Le procédé selon l'invention permet d'obtenir de manière parfaitement contrôlée avec une très grande activité, une très grande productivité, un très haut rendement, et une très haute sélectivité des polymères de PHA, de masse moléculaire élevée et avec une répartition étroite de la masse moléculaire.

En particulier, des polymères hétérotactiques de PHA avec une répartition étroite de masse moléculaire, ladite masse moléculaire étant une masse moléculaire moyenne en nombre élevée par exemple de 4 000 à 500 000 ont été obtenus grâce au procédé de l'invention, avec des activités et des productivités très élevées voire quantitatives, à température ambiante.

Le procédé selon l'invention permet notamment de contrôler la tacticité du polymère obtenu et de préparer préférentiellement des polymères essentiellement syndiotactiques à partir de lactones racémiques lorsque celles-ci existent.

L'invention concerne en outre un polymère de polyhydroxyalcanoate susceptible d'être obtenu par le procédé tel que décrit plus haut.

Ce Polymère de polyhydroxyalcanoate est avantageusement un PHA syndiotactique susceptible d'être obtenu par polymérisation d'une lactone (I) racémique.

Ladite lactone est de préférence la β-butyrolactone racémique ou la γ-valérolactone racémique.

Le polymère susceptible d'être obtenu par le procédé de l'invention, lorsqu'il est syndiotactique a généralement un degré de syndiotacticité de 70 à 99%.

Le polymère susceptible d'être obtenu par le procédé de l'invention a généralement une masse moléculaire moyenne en nombre de 4000 à 500 000, de préférence de 8000 ou 9000 à 170 000.

Le polymère susceptible d'être obtenu par le procédé de l'invention a généralement un indice de polydispersité PDI de 1,01 à 1,50, de préférence de 1,05 à 1,20.

L'invention concerne également une composition. de matière comprenant le polymère de polyhydroxyalcanoate décrit ci-dessus et d'autres ingrédients.

Avantageusement, cette composition est biodégradable ; auquel cas cette composition comprend généralement, en outre, un ou plusieurs polymères biodégradables différents du polyhydroxyalcanoate.

Dans les compositions selon l'invention, le polymère de polyhydroxyalcanoate est généralement présent à raison de 0,1 à 99,9%, de préférence 1 à 99%, de préférence encore 5 à 90%, mieux 10 à 80%, mieux encore 20 à 70%, par exemple 40 à 60%, notamment 50%, ou 55% en poids de poids total de la composition.

L'invention a trait aussi à l'utilisation du polymère susceptible d'être préparé par le procédé de l'invention ou de la composition telle que décrite plus haut dans des emballages notamment alimentaires.

L'invention concerne aussi l'utilisation du polymère susceptible d'être préparé par le procédé de l'invention ou de la composition telle que décrite plus haut, dans des dispositifs et procédés biomédicaux ou pour des applications biomédicales.

L'invention a trait en outre à l'utilisation du polymère susceptible d'être préparé par le procédé de l'invention ou de la composition telle que décrite plus haut, dans des fixations chirurgicales telles que des sutures, des agrafes et des plaques.

L'invention a enfin trait à l'utilisation du polymère susceptible d'être préparé par le procédé de l'invention ou de la composition telle que décrite plus haut, pour la libération contrôlée, retardée de médicaments.

L'invention va maintenant être décrite de manière détaillée dans la description qui suit faite à titre illustratif et non limitatif en relation avec les dessins joints dans lesquels :
- la figure 1 représente les régions carbonyle (a) et méthylène (b) des spectres en RMN du ¹³C (125 MHz, CDCl₃) du PHB préparé par polymérisation de la β-butyrolactone racémique avec le complexe (III) ;
- la figure 2 représente les thermogrammes obtenus en analyse calorimétrique différentielle à compensation de puissance (« Differential Scanning Calorimetry » en anglais) respectivement d'un polymère PHB syndiotactique à 85% (exemple 12, courbe a)) et d'un polymère PHB syndiotactique à 91% (exemple 2, courbe b)).

Dans la description qui suit on décrit le procédé selon l'invention en faisant essentiellement référence à la polymérisation de la β-butyrolactone racémique préférentiellement par le complexe de formule (III), mais il est bien évident que l'homme du métier comprendra que cette description peut s'appliquer aussi à la polymérisation d'autres lactones racémiques ou non avec le même complexe ou avec d'autres complexes décrits dans la présente.

Les initiateurs complexes mis en oeuvre dans le procédé selon l'invention sont préparés à partir de ligands diamino et alcoxy-amino bisphénol H₂L par exemple 1 à 6 qui sont généralement synthétisés par des doubles condensations de Mannich du phénol substitué correspondant, du formaldéhyde et de l'alcoxy-amine ou diamine appropriée selon le schéma 1 suivant :

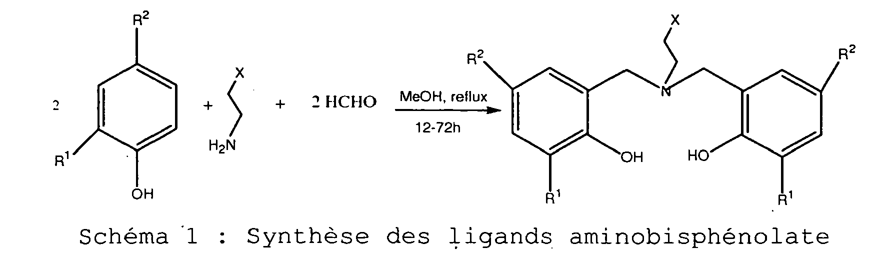

Dans le schéma 1, R₁, R₂ et X ont la signification déjà précisée plus haut.

Ils peuvent être isolés avec des rendements moyens à modérés sous la forme de poudres microcristallines. Certains de ces composés N,N-substitués sont difficiles à obtenir à cause de la formation de benzoxazines en tant que sous produits qui a lieu par cyclisation du produit intermédiaire N-substitué. On donne ci-après sur le schéma 2 la formule de ligands aminobisphénolates particuliers 1 à 6.

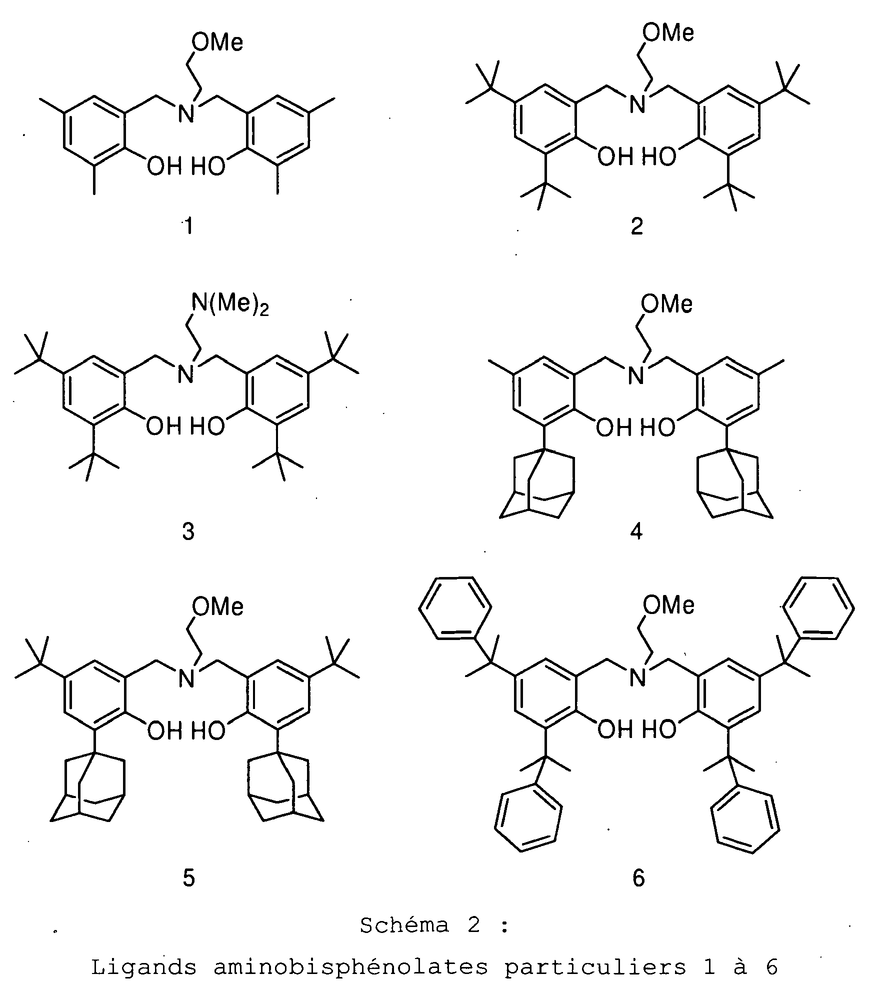

Des précurseurs de métaux du groupe 3 [MR'₃ (THF)₂] avec M = Y, La ; et R' = CH₂SiMe₃, N(SiHMe₂)₂ et [Na{N(SiMe₃)₂}₃] sont chacun traités avec un équivalent de ligand bisphénolate (et la formule générale apparaît dans le schéma 1), par exemple avec un équivalent de chacun des bisphénols H₂L 1 à 6 pour donner les complexes d'yttrium et de lanthane [(L)M(R')(THF)] 7 à 10, 12 à 14, 16 et 17, et [(L)Nd.{N(SiMe₃)₂}] 11 et 15 avec de bons rendements (schéma 3).

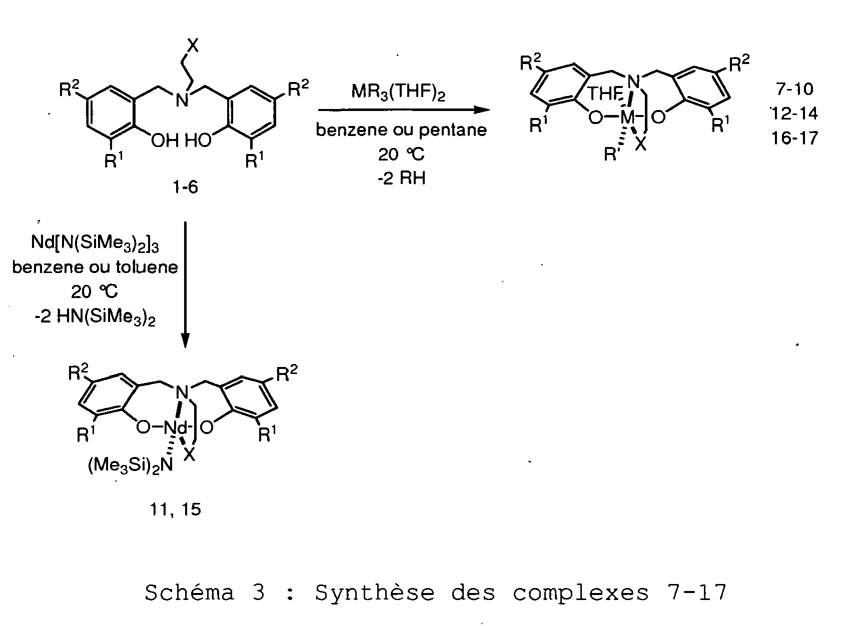

Le tableau 1 rassemble les significations des ligands, du métal, et des substituants pour les complexes 7 à 17.

**Tableau 1**

| Complexe | Métal | Ligand | R' |
|---|---|---|---|
| 7 | Y | 1 | N(SiMe₃)₂ |
| 8 | Y | 2 | N(SiHMe₂)₂ |
| 9 | Y | 2 | CH₂SiMe₃ |
| 10 | La | 2 | N(SiHMe₂)₂ |
| 11 | Nd | 2 | N(SiMe₃)₂ |
| 12 | Y | 3 | N(SiHMe₂)₂ |
| 13 | Y | 4 | N(SiHMe₂)₂ |
| 14 | La | 4 | N(SiHMe₂)₂ |
| 15 | Nd | 4 | N(SiMe₃)₂ |
| 16 | Y | 5 | N(SiHMe₂)₂ |
| 17 | Y | 6 | N(SiHMe₂)₂ |

Les complexes utilisés dans le procédé selon l'invention tels que le complexe (III) sont préparés à partir par exemple des complexes 7 à 17 par réaction de ceux-ci avec l'alcool adéquate de formule R³-OH (où R³ a la signification déjà donnée plus haut) à température ambiante dans un solvant tel que le [D₆] benzène ou le [D₈]THF (schéma 4). Un alcool préféré est l'isopropanol.

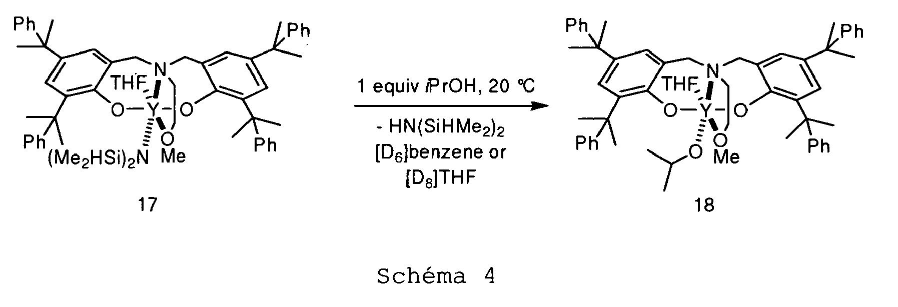

L'invention va maintenant être décrite dans la partie expérimentale et les exemples suivants qui ont trait à la synthèse des complexes aminobisphénoxy et à leur essai en polymérisation de la β-butyrolactone racémique.

### Conditions générales

La synthèse des complexes et les tests de polymérisation ont été effectués en boîte à gants. La verrerie utilisée est séchée à l'étuve à 120°C toute la nuit et refroidie sous vide avant utilisation. Les solvants utilisés (toluène, THF, pentane) sont distillés sur Na/K sous argon et sont dégazés plusieurs fois avant utilisation. Le benzène-d₆ est séché et dégazé avant utilisation. Les précurseurs métalliques Y (N (SiHMe₂)₂)₃THF₂, La (N (SiHMe₂)₂)₃THF₂, Nd (N (TMS)₂)₃ ont été synthétisés au laboratoire. La rac-butyrolactone est distillée deux fois sur CaH₂ sous vide, puis est conservée en boîte à gants. La synthèse des ligands a été faite à partir de réactifs commerciaux, ou à partir de réactifs synthétisés au préalable au laboratoire. Les ligands 2 et 3 ont été synthétisés selon les mêmes procédures déjà publiées.

### Synthèse du ligand bisaminophenoxy(2,4-dimethyl) (Composé 1)

Le formaldehyde (1.2 ml, 16 mmol) et la methoxyethylamine (0.52 ml, 6 mmol) sont mélangés à température ambiante. Le mélange est ensuite rajouté à une solution de 2,4-dimethylphénol (1.95 g, 16 mmol) dans le méthanol (4 ml). Le milieu réactionnel est ensuite porté à reflux 24h. Après que le mélange ait été refroidi, un précipité marron est observé, la solution est séparée du précipité, et celui-ci est redissous dans un minimum de méthanol, puis porté à reflux 2 h. Un précipité blanc est ainsi formé au fond de la solution. Le mélange est filtré sous vide, puis la poudre blanche obtenue est séchée sous vide. Le filtrat est laissé au réfrigérateur 24 h, des cristaux blancs sont obtenus (1,1 g, rdt 52%). RMN ¹H (200 MHz, CDCl₃, 25°C): δ 8.35 (s, 2 H ; ArO*H*), 6.85 (s, 2 H; Ar*H*), 6.67 (s, 2 H ; Ar*H*), 3.72 (s, 4 H ; ArC*H*₂), 3.58 (t, ³*J*(H, H) = 5.1 Hz, 2 H ; NCH₂C*H*₂O), 3.47 (s, 3 H ; OC*H*₃), 2.70 (t, ³*J*(H,H)= 4.9 Hz, 2 H; NC*H*₂CH₂O), 2.20 (s, 12 H; C*H*₃); ¹³C{¹H} RMN (75 MHz, [D₆]benzene, 25°C) : δ 152.84, 131.37, 121.24 (Ar-Cq), 127.68, 127.36, 125.15 (Ar-*C*H), 70.89 (NCH₂*C*H₂O), 58.17 (O*C*H₃) , 57.04 (NCH₂CH₂O) , 50.77 (*C*H₂Ar), 20.24, 16.03 (CH₃) ; HRMS (70 eV, EI): m/z calculé pour C₂₁H₂₉N₁O₃: 343.2147 ; trouvé : 343.2139 [*M*⁺].

### Synthèse du ligand bisaminophenoxy(2-adamantyl)-4-methyl (Composé 4)

Le formaldehyde (0.12 ml, 1.60 mmol) et la methoxyethylamine (0.052 ml, 0.60 mmol) sont mélangés à température ambiante. Le mélange est ensuite rajouté à une solution de (2-adamantyl-4-methyl)-phénol (0.38 g, 1.60 mmol) dans le méthanol (1.5 ml). Le milieu réactionnel est ensuite porté à reflux 48 h. Après que le mélange ait été refroidi, un précipité marron est observé, la solution est séparée du précipité, et celui-ci est redissous dans un minimum de méthanol, puis porté à reflux 2 h. Un précipité blanc est ainsi formé au fond de la solution. Le mélange est filtré sous vide, puis la poudre blanche obtenue est séchée sous vide (0.22 g, rdt 62%). RMN ¹H (CDCl₃, 200 MHz) : δ 8.37 (s, 2 H ; ArO*H*) , 6.93 (br s, 2 H ; Ar*H*), 6.69 (br s, 2 H ; Ar*H*), 3.67 (s, 4 H ; ArC*H*₂), 3.48 (br t, ³*J*(H,H) = 5.1 Hz, 2 H ; NCH₂C*H*₂O), 3.44 (s, 3 H ; OC*H*₃), 2.74 (t, ³*J*(H,H) = 5.1 Hz, 2 H ; NC*H*₂CH₂O), 2.22 (s, 6 H ; C*H*₃), 2.15 (s, 12 H ; C*H*₂-adamantyl), 2.04 (s, 6 H ; C*H*-adamantyl), 1.76 (s, 12 H ; C*H*₂-adamantyl); ¹³C{¹H} RMN (75 MHz, [D₆]benzène, 25°C) : δ 153.61, 137.39 (Ar-*Cq*), 128.69, 122.71 (Ar-*C*H), 71.07 (NCH₂*C*H₂O) , 58.13 (O*C*H₃), 57.44 (*C*H₂Ar), 51.28 (N*C*H₂CH₂O), 40.73 (adamantyl), 37.44 (adamantyl), 37.13 (adamantyl), 29.60 (adamantyl), 20.83 (CH₃) ; HRMS (4000 V, ESI) : *m*/*z* calculé pour C₃₉H₅₄N₁O₃: 584.4103 ; trouvé : 584.4107 [*M*⁺ + H].

### Synthèse du (2-adamantyl-4-tertiobutyl)-phénol

Dans un ballon sous argon, le 4-tertiobutylphénol (4 g ; 0.026 mol) est dissous dans un mélange Xylène/DMF (20 ml ; 10 ml), puis du sodium découpé en fin copeaux (0.5 g ; 0.026 mol) est ajouté sous argon. Une fois le sodium totalement dissous (coloration jaune de la solution), le 1-chloroadamantane (4.55 g ; 0.026 mol) est ajouté. Le mélange est maintenu sous agitation à 85°C, 24h. Après avoir laissé le milieu réactionnel revenir à température ambiante, le mélange réactionnel est dissous dans 100 ml d'éther, puis la phase aqueuse est extraite avec 100 ml d'une solution KOH à 10%. La phase aqueuse est lavée avec 3 fois 100 ml d'éther. Les phases éthérées sont réunies, puis lavées avec 100 ml d'eau et séchées sur MgSO₄. Le solvant est évaporé, et l'huile obtenue est séchée sous vide. 4.85 g de produit brut sont obtenus (rdt 65%), le produit est purifié par colonne chromatographique pour obtenir 1.84 g de (2-adamantyl-4-tertiobutyl)-phénol pur (Rdt 26%). RMN ¹H (CDCL₃, 200 MHz) : δ 7.25 (s, 1H, Phenyl), 7.09 (dd, J= 8 hz, 1H, phenyl), 6.59 (d, J= 8.1 Hz, 1H, phenyl), 4.59 (s, 1H, PhO*H*), 2.13 (bs, 9H, adamantyl), 1.78 (s, 6H, adamantyl), 1.29 (s, 9H, t-Bu).

### Synthèse du ligand bisaminophenoxy(2-adamantyl)-4-tertiobutyl (Composé 5)

Le formaldehyde (0.17 ml, 1.75 mmol) et la methoxyethylamine (0.057 ml, 0.65 mmol) sont mélangés à température ambiante. Le mélange est ensuite rajouté à une solution de (2-adamantyl-4-tertiobutyl)-phénol (0.5 g, 1.75 mmol) dans le méthanol (2 ml). Le milieu réactionnel est ensuite porté à reflux entre 24 et 48 h. Après que le mélange ait été refroidi, un précipité marron est observé, la solution est séparée du précipité, et celui-ci est redissous dans un minimum de méthanol, puis porté à reflux 2 h. Un précipité blanc est ainsi formé au fond de la solution. Le mélange est filtré sous vide, puis la poudre blanche obtenue est séchée sous vide (0.13 g, rdt 30%). Le filtrat est laissé au frigo 24 h, des cristaux blancs sont obtenus (rdt 56%), correspondant à la benzoxazine (2). Si la benzoxazine obtenue est mélangée avec 1 équivalent de (2-adamantyl-4-tertiobutyl)-phénol, et un équivalent de formaldéhyde, puis porté à reflux 24 h, le produit désiré est formé (52%). RMN ¹H (C₆D₆, 200 MHz) : δ 8.80 (s, 2 H ; PhO*H*), 7.48 (d, ⁴*J*(H, H) = 2.2 Hz, 2 H ; Ar*H*), 6.96 (d, ⁴*J*(H, H) = 2.2 Hz, 2 H ; Ar*H*), 3.53 (s, 4 H ; ArC*H*₂), 2.99 (br s, 5 H ; OC*H*₃ + NCH₂C*H*₂O), 2.50 (s, 12 H ; C*H*₂ adamantyl), 2.35 (t, ³*J*(H,H)= 2.2 Hz, 2 H ; NC*H*₂CH₂O), 2.18 (s, 6 H ; C*H* adamantyl), 1.92 (br m, 12 H ; C*H*₂ adamantyl), 1.37 (s, 18 H; C(C*H*₃)₃) ; ¹³C{¹H} RMN (75 MHz, [D₆]benzène, 25°C) : δ 153.39, 140.94, 136.59, 122.07 (Cq Ar), 124.76, 123.36 (Ar), 70.79 (NCH₂*C*H₂O), 58.12 (O*C*H₃), 57.63 (C*H*₂Ar), 50.93 (N*C*H₂CH₂O), 40.70 (adamantyl), 37.37 (adamantyl), 37.33 (adamantyl), 34.07 (adamantyl), 31.65 (*C*(CH₃)₃), 29.51 (C(*C*H₃)₃); HRMS (4000 V, ESI) : m/z calculé pour C₄₅H₆₆N₁O₃: 668.5042 ; trouvé : 668.5037 [*M*⁺ + H] .

### Synthèse du ligand bisaminophenoxy 2,4-dicumyle (Composé 6)

Le formaldehyde (1.12 ml, 15.1 mmol) et la methoxyethylamine (0.5 ml, 5.7 mmol) sont mélangés à température ambiante. Le mélange est ensuite rajouté à une solution de (2,4-dicumyl)-phénol (5 g, 15.1 mmol) dans le méthanol (4 ml). Le milieu réactionnel est ensuite porté à reflux 72h. Après que le mélange ait été refroidi, un précipité blanc est observé, la solution est séparée du précipité, et celui-ci est redissous dans un minimum de méthanol, puis porté à reflux 24 h. Un précipité blanc est ainsi formé au fond de la solution. Le mélange est filtré sous vide, puis la poudre blanche obtenue est séchée sous vide (rdt 30%). RMN ¹H (C₆D₆, 200 MHz) : δ 8.11 (s, 2 H ; PhO*H*) , 7.47 (d, ⁴*J*(H,H) = 1.8 Hz, 2 H ; Ar*H*), 7.33 (t, ³*J*(H,H) = 7.7 Hz, 16 H ; phenyl.cumyl), 7.06 (m, 4 H ; phenyl cumyl) 6.83 (d, ³*J*(H,H) = 1.3 Hz, 2 H ; Ar*H*), 3.28 (s, 4 H ; ArC*H*₂), 2.68 (br s, 5 H ; OC*H*₃ + NCH₂C*H*₂O), 2.15 (t, ³*J*(H,H) = 5.1 Hz, 2 H ; NC*H*₂CH₂O) , 1.75 (s, 12 H ; C*H*₃ cumyl), 1.68 (s, 12 H; CH₃ cumyl); ¹³C{¹H} RMN (75 MHz, [D₆]benzène, 25°C) : δ 153.52, 152.16, 151.70, 141.29, 136.51 (*Cq* Ar) , 128.96, 128. 40, 127.63, 126.80, 126.32, 123. 57 (Ar) , 71.57 (s, NCH₂ *C*H₂O) 58.68 (O*C*H₃) , 57.60 (*C*H₂Ar), 51.67 (s, N*C*H₂CH₂O), 43.26 (*C*(CH₃)₂), 42.95 (*C*(CH₃)₂), 31.82 (CH₃), 30.17 (*C*H₃) ; HRMS (4000 V, ESI) : m/z calculé pour C₅₃H₆₂N₁O₃: 760.4729 ; trouvé : 760.4726 [*M*⁺ +H].

### Reaction de Y [N (SiMe₃)₂]₃ avec 1. Generation de "7"

Une solution de 1 (53.4 mg, 0.105 mmol) dans le toluène (5 mL) est ajouté à une solution de Y[N(SiMe₃)₂]₃ (60.0 mg, 0.105 mmol) dans le toluène (5 mL) à température ambiante. Le mélange est agité pendant 12 h à température ambiante et 2 h à 60°C. Puis, la solution est évaporée sous vide. Le solide est lavé avec un peu de pentane à froid, puis séché sous vide pour donner une poudre blanche (43.9 mg, 67%). RMN¹H (200 MHz, [D₆]benzène, 25°C) pics caractéristiques: δ 5.60 (d, ²*J*(H, H) = 11.7 Hz, 0.5 H ; ArC*H*₂), 5.45 (d, ²*J*(H,H) = 11.9 9 Hz, 0.5 H ; ArC*H*₂), 5.22 (d, ²*J*(H,H) = 12.4 Hz, 0.8 H ; ArC*H*₂), 5.09 (dd, ²*J*(H,H) = 4.0 Hz, ²*J*(H,H) = 11.3 Hz, 0.8 H ; ArC*H*₂), 4.71 (d, ²*J*(H,H) = 12.4 Hz, 2 H ; ArC*H*₂) , 4.61 (d, ²*J*(H,H) = 12.4 Hz, 2 H ; ArC*H*₂) , 4.03 (d, ²*J*(H,H) = 11.9 Hz, 0.6 H ; ArC*H*₂) , 3.58 (d, ²*J*(H, H) = 12.4 Hz, 0.6 H ; ArC*H*₂), 0.37 (br s, 18 H ; NSi(C*H*₃)₃), 0.31 (s, 16 H ; NSi(C*H*₃)₃). Les pic pour HNSi(C*H*₃)₃ sont aussi observés (δ 0.12 (s, 92H), tandis que le pic du ArO*H* disparait. le complexe est directement engagé en catalyse.

### Synthèse du complexe 8

Une solution de 2 (0.153 g, 0.30 mmol) dans le pentane (5 mL) est ajoutée à une solution de Y [N (SiHMe₂)₂]₃(THF)₂ (0.189 g, 0.30 mmol) dans le pentane (5 mL) à température ambiante. Le mélange est agité pendant 12 h à température ambiante,un précipité blanc est alors obtenu. Pour assurer un conversion complète, le mélange est encore laissé sous agitation 10 h de plus. Le solide est alors filtré pour donner une poudre blanche 8 (0.163 g, 67%). RMN ¹H (300 MHz, [D₆]benzène, 25°C): δ 7.60 (d, ⁴*J*(H,H)= 2.5 Hz, 2 H ; Ar*H*), 7.10 (d, ⁴*J*(H,H) = 2.3 Hz, 2 H ; Ar*H*), 5.14 (m, 2 H ; Si*H*) , 3.87 (d, ²*J*(H,H) = 12.5 Hz, 2 H ; chevauchement avec le signal du THF, ArC*H*₂), 3.84 (br m, 4 H ; α-C*H*₂ THF), 2.97 (d, ²*J*(H,H) = 12.5 Hz, 2 H ; ArC*H*₂) , 2.84 (s, 3 H ; OC*H*₃) , 2.71 (t, ³*J*(H,H) = 5.2 Hz, 2 H ; NCH₂C*H*₂O) , 2.31 (t, ³*J*(H,H) = 5.2 Hz, 2 H ; NC*H*₂CH₂O), 1.79 (s, 18 H; C(C*H*₃)₃), 1.47 (s, 18 H; C(C*H*₃)₃), 1.18 (m, 4 H ; β-CH₂ THF), 0.49 (d, ⁴*J*(H,H) = 3.0 Hz, 12 H ; HSi (C*H*₃)₂) ; RMN ¹H (300 MHz, [D₈]THF, 25°C) : δ 7.19 (d, ⁴*J*(H,H) = 2.5 Hz, 2 H ; Ar*H*), 6.91 (br s, 2 H ; Ar*H*), 4.90 (m, 2 H; Si*H*), 4.00 (d, ²*J*(H,H) = 12.5 Hz, 2 H ; ArC*H*₂), 3.58 (m, 4 H; α-CH₂ THF, chevauchement avec les résonances du solvant), 3.26 (m, 7 H; ArC*H*₂, OC*H*₃, NCH₂C*H*₂O), 2.70 (t, ³*J*(H,H) = 5.2 Hz, 2 H ; NC*H*₂CH₂O), 1.73 (m, 4 H ; β-C*H*₂ THF, chevauchement avec les résonances du solvant), 1.48 (s, 18 H ; C(C*H*₃)₃), 1.26 (s, 18 H ; C (C*H*₃)₃), 0.15 (d, ⁴*J*(H,H) = 3.0 Hz, 12 H ; HSi(C*H*₃)₂); ¹³C{¹H} RMN (75 MHz, [D₆]benzène, 25°C): δ 161.38, 136.53, 125.40, 124.11 (Ar), 73.11 (NCH₂*C*H₂O), 70.96 (α-*C*H₂ THF), 64.54 (*C*H₂Ar) , 60.43 (OCH₃), 49.57 (N*C*H₂CH₂O), 35.46 (*C*(CH₃)₃), 34.04 (*C*(CH₃)₃), 32.06 (C(CH₃)₃), 30.30 (C(*C*H₃)₃), 24.95 (s, β-*C*H₂ THF), 4.22 (HSi(*C*H₃)₂) ; analyse élémentaire calculée (%) pour C₄₁H₇₃N₂O₄Si₂Y: C 61.32, H 9.17, N 3.49 ; trouvée : C 61.74, H 9.36, N 3.36.

### Synthèse du complexe 9

Une solution de 2 (0.153 g, 0.30 mmol) dans le pentane (5 mL) est ajoutée à une solution de Y(CH*₂*SiMe₃)₃(THF)₂ (0.148 g, 0.30 mmol) dans le pentane (5 mL) à 0°C. Le mélange est agité pendant 2 h à 0°C. Puis, la solution est évaporée sous vide. Le solide est lavé avec un peu de pentane à froid, puis séché sous vide pour donner une poudre blanche 9 (0.16 g, 70%). RMN ¹H (300 MHz, [D₆]benzène, 25°C): δ 7.59 (d, ⁴*J*(H,H) = 2.5 Hz, 2 H ; Ar*H*), 7.08 (d, ⁴*J*(H,H) = 2.5 Hz, 2 H ; Ar*H*), 3.87 (br m, 4 H ; α-C*H*₂ THF), 3.76 (d, ²*J*(H,H) = 12.5 Hz, 2 H ; ArC*H*₂) , 2.92 (d, ²*J*(H,H) = 12.5 Hz, 2 H ; ArC*H*₂) , 2.88 (s, 3 H ; OC*H*₃) , 2.44 (t, ³*J*(H,H) = 5.3 Hz, 2 H ; NCH₂C*H*₂O) , 2.21 (t, ³*J*(H,H) = 5.3 Hz, 2 H ; NC*H*₂CH₂O), 1.80 (s, 18 H ; C(C*H*₃)₃), 1.46 (s, 18 H ; C(C*H*₃)₃) , 1.27 (br m, 4 H ; βC*H*₂ THF), 0.49 (s, 9 H ; Si(C*H*₃)₃), 0.40 (d, ²*J*(Y-H) = 3.1 Hz, 2 H ; C*H*₂Si(CH₃)₃)); ¹³C{¹H} RMN (75 MHz, [D₆]benzene, 25°C): δ 161.38, 136.59, 136.40, 125.40, 124.21, 123.90 (Ar), 73.84 (NCH₂*C*H₂O), 70.66 (α-CH₂ THF) , 64.65 (*C*H₂Ar), 61.09 (OCH₃), 49.09 (N*C*H₂CH₂O), 35.39 (*C*(CH₃)₃), 34.04 (C(CH₃)₃), 32.05 (C(*C*H₃)₃), 30.11 (C(CH₃)₃), 24.92 (β-CH₂ THF), 24.70 (d, ¹*J*(C-Y) = 46.4 Hz ; Y*C*H₂), 4.64 (Si(CH₃)₃); analyse élémentaire calculée (%) pour C₄₁H₇₀NO₄SiY : C 64.97, H 9.31, N 1.85 ; trouvée : C 65.11, H 9.65, N 1.74.

### Synthèse du complexe 10

Une solution de 2 (0.204 g, 0.40 mmol) dans le pentane (5 mL) est ajoutée à une solution de La[N(SiHMe₂)₂]₃(THF)₂ (0.272 g, 0.40 mmol) dans le pentane (5 mL) à température ambiante. Le mélange est agité pendant 24 h à température ambiante, puis, la solution est évaporée sous vide. Le solide est lavé avec un peu de pentane à froid, puis séché sous vide pour donner une poudre blanche 10 (0.18 g, 92%). RMN ¹H (300 MHz, [D₆]benzène, 25°C): δ 7.57 (d, ⁴*J*(H,H) = 2.3 Hz, 2 H ; ArH), 7.11 (d, ⁴*J*(H,H) = 2.5 Hz, 2 H; ArH), 5.25 (m, 2 H; Si*H*), 3.76 (br m, 4 H; α-C*H*₂ THF), 3.58 (d, ²*J*(H,H) = 12.5 Hz, 2 H ; ArC*H*₂), 3.32 (d, ²*J*(H,H) = 12.5 Hz, 2 H ; ArC*H*₂) , 3.07 (s, 3 H ; OC*H*₃), 2.77 (t, ³*J*(H,H) = 5.2 Hz, 2 H ; NCH₂C*H*₂O), 2.27 (m, 2 H; NC*H*₂CH₂O), 1.73 (s, 18 H; C(C*H*₃)₃), 1.44 (s, 18 H ; C(C*H*₃)₃), 1.22 (m, 4 H; βC*H*₂ THF) , 0.48 (d, ⁴*J*(H,H) = 3.0 Hz, 12 H ; Si(C*H*₃)₂); ¹³C{¹H} RMN (75 MHz, [D₆]benzène, 25°C): δ 161.8, 136.6, 135.6, 125.8, 124.3, 123.9 (Ar), 71.9 (NCH₂*C*H₂O), 69.9 (α-CH₂ THF), 61.6 (CH₂Ar), 60.9 (OCH₃), 50.7 (N*C*H₂CH₂O), 35.3 (*C*(CH₃)₃), 34.0 (*C*(CH₃)₃), 32.0 (C(*C*H₃)₃), 30.1 (C(*C*H₃)₃), 25.0 (β-*C*H₂ THF), 3.5 (Si(*C*H₃)₂); analyse élémentaire calculée (%) pour C₄₁H₇₃N₂O₄LaSi₂: C 57.72, H 8.62, N 3.28 ; trouvée : C 57.91, H 9.03, N 3.18.

### Synthèse du complexe 11

L'amidure de néodyme Nd (N (SiMe₃)₂)₃ (10 mg ; 0.016 mmol) est dissous en tube RMN dans le benzène deutérié (2 mL), puis un équivalent de ligand 2 (9.3 mg ; 0.016 mmol) est ajouté. La solution est laissée toute la nuit à température ambiante. Puis la solution est portée à 60°C plusieurs heures. Une fois le solvant évaporé, le complexe est directement engagé en catalyse.

Reaction en tube RMN de Y[N(SiHMe₂)₂]₃(THF)₂avec 3. Generation de 12.

A une solution de Y[N(SiHMe₂)₂]₃(THF)₂ (17.1 mg, 0.030 mmol) dans le toluene[D₈] (ca. 0.5 mL) en tube RMN est ajouté un équivalent de ligand 3 (15.9 mg, 0.030 mmol) à température ambiante. Après 1 h, la réaction est suivie par spectroscopie RMN ¹H qui indique une conversion complète du précurseur d'yttrium et du ligand de départ, avec libération de 2 equiv d'amine libre HN(SiHMe₂)₂. A cause du comportement fluxionnel à température ambiante du produit formé, des informations RMN n'ont pu être obtenues qu'à 60°C. pics caractéristiques RMN ¹H (500 MHz, [D₈]toluène, 60°C): δ espèces principales 7.48 (d, ⁴*J*(H,H) = 2.7 Hz, 4 H ; aryl), 6.88 (br s, 4 H; aryl), 5.00 (m, 1 H; Si*H*(CH₃)₂); espèces secondaires 7.49 (d, ⁴*J*(H,H) = 3.2 Hz, 4 H ; aryl), 6.81 (d, ⁴*J*(H,H) = 3.2 Hz, 4 H ; aryl), 4.90 (m, 1 H ; Si*H*(CH₃)₂).

### Synthèse du complexe 13.

L'amidure d'Yttrium Y(N(SiHMe₂)₂)₃.THF₂ (20 mg ; 0.033 mmol) est dissous en tube RMN dans le benzène deutérié (2 mL), puis un équivalent de ligand 4 (20.4 mg ; 0.033 mmol) est ajouté. La solution est laissée toute la nuit à température ambiante. RMN ¹H (C₆D₆, 300MHz) : δ 7.23 (d, ⁴*J*(H,H) = 2.2 Hz, 2 H ; Ar*H*), 6.18 (d, ⁴*J*(H,H) = 2. 4 Hz, 2 H ; Ar*H*), 5.21 (m, 2 H ; Si*H*(CH₃)₂), 3.93 (m, 4 H ; α-C*H*₂ THF), 3.74 (d, ²*J*(H,H) = 11.9 Hz, 2 H ; ArC*H*₂), 2.98 (d, ²*J*(H,H) = 12.4 Hz, 2 H ; ArC*H*₂), 2.84 (t, ³*J*(H,H) = 4.8 Hz, 4H ; NCH₂C*H*₂O), 2.46 (s, 12 H; adamantyl), 2.40 (s, 6 H ; C*H*₃), 2.26 (s, 9 H ; adamantyl + OC*H*₃) , 2.03 (d, ²*J*(H,H) = 11.7 Hz, 6 H ; adamantyl), 1.92 (d, ²*J*(H, H) = 11.3 Hz, 6 H ; adamantyl), 1.23 (m, 4 H; α -C*H*₂ THF), 0.49 (d, ⁴*J*(H,H) = 2.9 Hz, 12 H ; SiH (C*H*₃)₂) ; ¹³C{¹H} RMN (75 MHz, [D₆]benzène, 25°C): δ 162.39, 129.78, 128.51 (Ar), 72. 41 (NCH₂CH₂O), 69.60 (α -*C*H₂ THF), 63.38 (*C*H₂Ar), 58.43 (N*C*H₂CH₂O), 48.35 (O*C*H₃), 40.64 (adamantyl), 37.40 (adamantyl), 37.06 (adamantyl), 29.09 (adamantyl), 24.36 (α -*C*H₂ THF), 20.33 (*C*H₃), 3.46 (Si(CH₃)₂) ; analyse élémentaire calculée (%) pour C₄₇H₇₃N₂O₄Si₂Y: C 64.50, H 8.41, N 3.20 ; trouvée : C 64.72, H 8.27, N 3.18.

### Synthèse du complexe [L4LaN(SiHMe₂)₂] 14 (Schéma 2)

L'amidure de lanthane La (N (SiHMe₂) ₂) ₃THF₂ (10 mg ; 0.016 mmol) est dissous en tube RMN dans le benzène deutérié (2 mL), puis un équivalent de ligand 4 (10.5 mg ; 0.016 mmol) est ajouté. La solution est laissée toute la nuit à température ambiante. RMN ¹H (C₆D₆, 500 MHz) : δ 7.20 (br s, 2 H; Ar*H*) , 6.80 (br s, 2 H; Ar*H*), 5.34 (m, 2 H; Si*H*(CH₃)₂), 3.69 (m, 4 H; α-C*H*₂ THF), 3. 38 (m, 2 H; ArC*H*₂), 3.17 (s, 4 H; NC*H*₂C*H*₂O), 2.82 (m, 2 H; ArC*H*₂) , 2.43 (s, 12 H; adamantyl), 2.40 (s, 6 H; C*H*₃), 2.19 (s, 9 H; adamantyl + OC*H*₃) , 1.99 (d, ²*J*(H,H) = 12.8 Hz, 6 H; adamanityl), 1.91(d, ²*J*(H,H) = 12.8 Hz, 6 H; adamantyl), 1.36 (m, 4 H; β-C*H*₂ THF), 0.49 (d, ⁴*J*(H,H) = 2.9 Hz, 12 H; SiH(*C*H₃)₂); ¹³C{¹H} RMN (125 MHz, [D₆]benzène, 25°C): δ 162.30, 129.70, 128.00 (Ar), 71.29 (CH₂Ar), 69.08 (α-CH₂ THF), 61.48 (N*C*H₂CH₂O), 48.35 (O*C*H₃), 40.83 (adamantyl), 37.64 (adamantyl), 37.12 (adamantyl), 29.75 (adamantyl), 25.36 (β-*C*H₂ THF), 20.98 (CH₃), 3.44 (Si(*C*H₃)₂).

### Synthèse du complexe 15

Une solution du complexe 4 (9.3 mg, 0.016 mmol) dans le benzène (1 mL) est ajoutée à une solution de Nd[N(SiMe₃)₂]₃ (10.0 mg, 0.016 mmol) dans le benzene (1.5 mL) à température ambiante. La solution est agitée pendant 12h à température ambiante puis 2 h à 60°C. Les composés volatils sont alors retirés sous vide puis le résidu est lavé avec une quantité minimale de pentane froid, séché sous vide, pour donner 15 sous la forme d'une poudre bleu clair. Le produit a été directement utilisé en polymérisation.

### Synthèse du complexe 16

L'amidure d'Yttrium Y(N(SiHMe₂)₂)₃.THF₂ (10 mg ; 0.016mol) est dissous en tube RMN dans le benzène deutérié (2 mL), puis un équivalent de ligand 5 (10.5 mg ; 0.016mmol) est ajouté. La solution est laissée toute la nuit à température ambiante. RMN ¹H (C₆D₆, 500MHz) : 5 7.54 (d, ⁴*J*(H,H) = 2.3 Hz, 2 H; Ar*H*) , 7.08 (d, ⁴*J*(H,H) = 2.2 Hz, 2 H; Ar*H*), 5.20 (m, 2 H; Si*H*Me₂), 3.82 (m, 4 H; α -C*H*₂ THF), 3.72 (br s, 2 H; ArC*H*₂), 3.11 (d, ²*J*(H,H) = 12.4 Hz, 2 H; ArC*H*₂) , 2.82 (t, ³*J*(H,H) = 4.5 Hz, 4 H; NC*H*₂C*H*₂O), 2.53 (s, 12 H; adamantyl), 2.34 (s, 3 H; OC*H*₃), 2.28 (s, 6 H; adamantyl), 2.08 (d, ²*J*(H,H) = 9.2 Hz, 6 H; adamantyl), 1.92 (d, ²*J*(H,H) = 10.9 Hz, 6 H; adamantyl), 1.47 (s, 18 H; C(C*H*₃)₃), 1.23 (m, 4 H; THF), 0.49 (d, ⁴*J*(H,H) = 2.9 Hz, 12 H; SiH(C*H*₃)₂); ¹³C{¹H} RMN (75 MHz, [D₆]benzène, 25°C): δ 162.39, 125.51, 124.21 (Ar), 72.41 (NCH₂CH₂O), 69.70 (α -C*H*₂ THF), 63.45 (*C*H₂Ar), 48.35 (OCH₃), 41.14 (adamantyl), 37.57 (adamantyl), 37.48 (adamantyl), 32.42 (C(*C*H₃)₃), 29.67 (adamantyl), 24.36 (β-*C*H₂ THF), 4.12 (Si(*C*H₃)₂) ; analyse élémentaire calculée (%) pour C₅₃H₈₅N₂O₄Si₂Y: C 66.36, H 8.93, N 2.92; trouvée : C 66.24, H 8.73, N 2.57.

### Synthèse du complexe 17

L'amidure d'Yttrium Y(N(SiHMe₂)₂)₃.THF₂ (82.6 mg ; 0.13 mmol) est dissous en tube de shlenck dans le pentane (5 mL), puis un équivalent de ligand 6 (100 mg ; 0.13 mol) est ajouté en solution dans le toluène (5 ml). La solution est laissée toute la nuit à température ambiante. Le solvant est évaporé sous vide et un solide blanc 95 mg est obtenu (rdt 68%). RMN ¹H (C₆D₆, 500MHz) : δ 7.53 (br s, 2 H; Ar*H*), 7.41 (d, ³*J*(H, H) = 7.1 Hz, 8 H; cumyl), 7.22 (t, ³*J*(H, H) = 7.7 Hz, 4 H; cumyl), 7.13 (m, 6 H; cumyl), 6.95 (t, ³*J*(H,H) = 4.5 Hz, 2 H; cumyl), 6.78 (br s, 2 H; ArH), 4.80 (br s, 2 H; Si*H*(CH₃)₂) , 3.35 (br s, 2 H; ArC*H*₂) , 2.93 (m, 4 H; THF), 2.80 (br s, 3 H; OC*H*₃), 2.69 (br s, 2 H; ArC*H*₂), 2.48 (br s, 2 H; NCH₂C*H*₂O) , 2.16 (br s, 6 H; C*H*₃ cumyl), 2.04 4 (br s, 2 H; NC*H*₂CH₂O) , 1.77 (br s, 12 H; CH₃ cumyl), 1.74 (br s, 6 H; CH₃ cumyl), 1.10 (m, 4 H; THF), 0.48 (br s, 12 H; SiH (C*H*₃)₂) ; ¹³C{¹H} RMN (75 MHz, [D₆]benzène, 25°C) : δ 161.34 (Cq, Ar) , 152.28 (Cq, cumyl), 137.64, 135.79 (cumyl-*C*), 128.11, 127.17 (Ar), 127.17, 126.21 (cumyl-*C*), 72.65 (NCH₂*C*H₂O), 69.70 (α-*C*H₂ THF) , 63.81 (CH₂Ar), 59.87 (OCH₃), 47.14 (N*C*H₂CH₂O), 42.49 (*C*(CH₃)₂), 31.46 (C(*C*H₃)₂), 27.58 (C(*C*H₃)₂), 24.81 (β-*C*H₂ THF) , 4.40 (Si(*C*H₃)₂) ; analyse élémentaire calculée (%) pour C₆₁H₈₁N₂O₄Si₂Y: C 69.68, H 7.77, N 2.66; trouvée : C 69.57, H 7.83, N 2.74.

### Synthèse du complexe 18 en tube RMN

Un équivalent d'isopropanol sec (0.67 µL, 0.0152 mmol)est ajouté à la µ-seringue à une solution du complexe 17 (16.0 mg, 0.0152 mmol) dans le THF-d₈ en tube RMN. Le tube est vigoureusement agité et laissé à température ambiante une dizaine de minutes. La réaction est suivie par RMN ¹H, indiquant la formation complète du complexe 18. RMN ¹H (THF-d⁸, 500MHz) : δ 7.25 (m, 4 H; cumyl), 7.20 (m, 8 H; cumyl), 7.06 (m, 8 H; cumyl), 6.92 (br s, 2 H; ArH) , 6.69 (br s, 2 H; Ar*H*), 4.20 (br m, 1 H; C*H*(CH₃)₂), 3.71 (d, ²*J*(H,H) = 12.0 Hz, 2 H; ArC*H*₂), 3.58 (m, 4 H; THF), 3.40 (br s, 3 H; OC*H*₃), 3.01 (br s, 2 H; NCH₂C*H*₂O), 2.86 (d, ²*J*(H,H) = 12.0 Hz, 2 H; ArC*H*₂), 2.16 (br s, 2 H; NC*H*₂CH₂O), 2.04 (br s, 6 H; C*H*₃ cumyl) , 1.77 (m, 4 H; THF) , 1.60 (br s, 12 H; C*H*₃ cumyl), 1.42 (s, 6 H; C*H*₃ cumyl), 1.15 (d, ³*J*(H, H) = 5.9 Hz, 6 H; CH(C*H*₃)₂) ; 13C{¹H} RMN (75 MHz, [D₈]THF, 25°C): δ 162.14 (*C*q Ar), 152.07 (*C*q cumyl), 134.36 (cumyl-*C*), 127.38, 126.55 125.70, 125.08, 123.46 (Ar), 72.77 (N*C*H₂CH₂O), 69.70 (α-C*H*₂ THF), 65.63 (C*H*(CH₃)₂) 63.72 (*C*H₂Ar), 61.15 (O*C*H₃), 49.75 (N*C*H₂CH₂O), 32.37 (*C*(CH₃)₂), 30.78 (C(*C*H₃)₂), 28.03 (CH(*C*H₃)₂), 26.27 (C(*C*H₃)₂), 24.81 (β-*C*H₂ THF).

Les complexes de la formule (II) mentionnée plus haut préparés tels que cela est décrit ci-dessous, et, en particulier, le complexe (18), sont des initiateurs actifs pour la polymérisation par ouverture de chaîne de la BBL racémique.

On décrit ci-après une façon type de réaliser la polymérisation de la β-butyrolactone.

Le complexe tel que le complexe 18 est dissous dans le toluène (0.6 mL) ou le THF (0.6 mL), puis 200 équivalents de *rac*-β-butyrolactone sont ajoutés à la seringue. Le mélange est laissé sous agitation à température ambiante, la formation d'un gel est rapidement observée. La conversion est suivie par RMN. La réaction est terminée par ajout de quelques gouttes d'une solution de méthanol acidifié (solution à 10% vol. de HCl), puis solubilisation dans le chloroforme (10 mL). Le polymère est précipité dans le méthanol (100 mL) à température ambiante. La solution est filtrée et le produit est séché sous vide. ( cf. tableau).

Des données de polymérisation représentatives sont par ailleurs regroupées dans le tableau 2.

La polymérisation de la BBL racémique avec le complexe (I) de métal du groupe 3 se déroule généralement rapidement à 20°C.

Certains des polymères produits ont des répartitions de masse moléculaire étroites et des valeurs de masses moléculaires moyennes en nombre (Mₙ) proche des valeurs théoriques (calculées en supposant que chaque groupe isoproxy initie la polymérisation).

Ces données indiquent que la polymérisation se déroule de manière vivante, c'est-à-dire sans réactions secondaires significatives.

De nombreux échantillons de la PHB sont totalement insolubles dans le THF. Ces échantillons sont, cependant, facilement dissous à température ambiante dans les solvants chlorés, tels que le dichlorométhane ou le chloroforme.

On a tout d'abord observé une forte influence du solvant, en comprenant les polymérisations dans le toluène, le THF et le chlorobenzène.

Pour un rapport [BBL]/[Y], Y désignant le complexe, notamment d'yttrium, la polymérisation dans le toluène et le chlorobenzène a atteint 97% de conversion èn moins d'une minute (exemples 1 et 3), tandis que la polymérisation dans le THF atteint 98% de conversion en l'espace de 2 heures (exemple 4).

En conséquence, on a réalisé toutes les autres réactions de polymérisation dans le toluène.

En utilisant un initiateur alcoolate de β-diiminate de zinc dans les mêmes conditions réactionnelles, COATES et AL. [13], ont indiqué qu'ils parvenaient à effectuer la polymérisation par ouverture de cycle de 200 équivalents de BBL racémique en l'espace d'une heure.

Plusieurs réactions ont été réalisées avec le complexe de l'yttrium (III) en modifiant le rapport monomère sur métal. Par exemple, là conversion complète de 400 équivalents de BBL racémique a été obtenue en une 1 minute à température ambiante (vitesse de rotation du catalyseur, TOF = 24 000 h⁻¹ ; exemple 5).

Il est, en particulier, à noter que l'alcoolate (isopropylate) d'yttrium (III) peut polymériser 1 000 équivalents en 5 minutes à 20°C dans la butyrolactone pure (TOF = 12 000 h⁻¹ ; exemple 14) et 2 000 équivalents à concentration élevée en moins de 20 minutes (TOF = 6 000 h⁻¹ ; exemple 15).

De plus, les expériences de polymérisation avec le complexe 1 en présence de trois équivalents d'isopropanol montrent qu'un échange alcoolate/isopropanol se produit durant le processus de propagation de la chaîne.

Après la consommation d'un équivalent d'isopropanol pour la formation de l'alcoolate d'yttrium, l'alcool libre en excès remplace les chaînes polymères alcoolates en croissance et agit en tant qu'agent de transfert de chaîne (exemple 10).

Un but important qui est poursuivi dans la présente invention est d'étudier l'incorporation asymétrique de la BBL dans la chaîne principale du polymère.

A ces fins, la spectroscopie en RMN du ¹³C est applicable à la détermination de la stéréochimie des PHB par inspection de la région des carbonyles (pour les "diades") et méthylène (pour les "triades") des spectres de RMN¹³C des polymères.

L'analyse microstructurale des différents PHB formés à partir de la BBL racémique révèle que le complexe (II) de métal du groupe 3 exerce, à température ambiante, une influence significative sur la tacticité du polymère formé selon le schéma 2 ci-dessous :

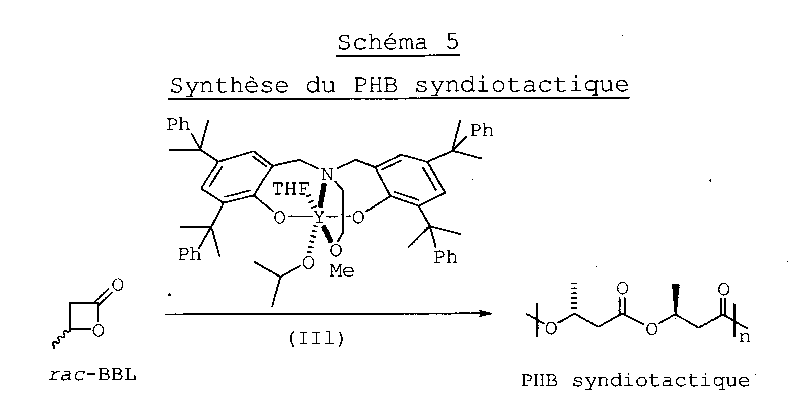

Sur la base des attributions précédentes des pics de la RMN¹³C de PHB, les signaux amont ("upfield") et aval ("downfield") de la région carbonyle correspondent respectivement aux séquences diades (R-R et S-S) méso(m), et aux séquences diades (R-S et S-R) racémiques (r) .

Il est intéressant de noter que deux séquences stéréochimiques (r) sont différentes à cause de l'effet de "directionalité" de la liaison ester, et sont clairement séparées.

Les PHB's préparées par le procédé de l'invention révèlent une forte contribution des diades r (δ 169,15 ppm) qui sont révélatrices d'un PHB fortement enrichi en forme syndiotactique (figure la).

On a détecté jusqu'à 94% de diades r (Pᵣ = 0,94), en fonction des conditions réactionnelles utilisées.

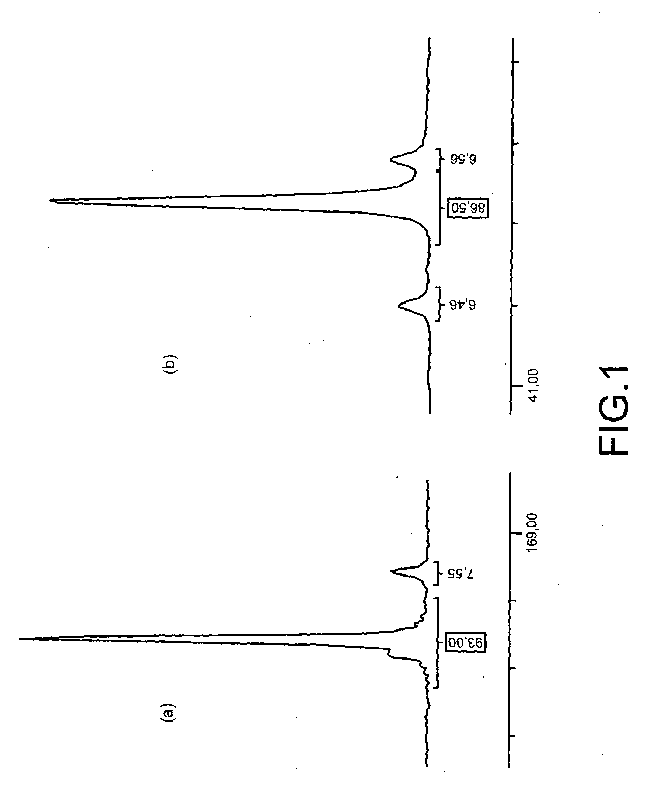

L'observation de l'expansion de la région des méthylènes sur la figure 1b montre trois pics (le quatrième pic théorique est trop peu intense pour être observé), qui correspondent à la sensibilité triade.

Sur la base des attributions précédentes, on a déterminé que la résonance la plus intense à 40,80 ppm correspond à la triade (rr) avec une intégration relative de 86%. Il y a lieu de noter que la séparation des deux séquences stéréochimiques a été observée lors de l'expansion des régions des signaux des carbones méthyle (séparée en deux pics) et méthine (séparée en trois pics).

Parmi les signaux des carbones méthyle et méthine, les séquences diades et triades ont été attribuées et sont en bon accord avec les signaux des carbones carbonyle et méthylène.

On a observé des différences dans la microstructure de la PHB en fonction de la nature du solvant utilisé.

Alors que la polymérisation de la BBL racémique dans le toluène et le chlorobenzène donne un PHB avec près de 90% d'enchaînements syndiotactiques (Pᵣ = 0,90), la polymérisation dans le THF donne seulement 83% de syndiotacticité (Pᵣ = 0,83).

Nous nous sommes également intéressés à l'influence de la température. Il est évident que le degré de stéréorégulation syndiotactique diminue au fur et à mesure que la température de polymérisation augmente comme l'on pouvait le prévoir.

A de faibles températures (-20°C), la polymérisation se déroule lentement, mais le PHB obtenu a un fort degré de syndiotacticité (Pᵣ = 0,94 ; exemple 8). En revanche, à des températures plus élevées la polycondensation se déroule plus rapidement (800 équivalents convertis en 3 minutes à 50°C au lieu de 10 minutes à température ambiante), comme l'on pouvait le prévoir, et fournit des PHB avec seulement 85% d'enchaînement syndiotactique et probablement avec une polydispersité plus large (exemples 11 et 12).

Les analyses thermiques de certains échantillons ont été réalisées et montrent la forte influence de la stéréochimie sur les propriétés physiques du PHB.

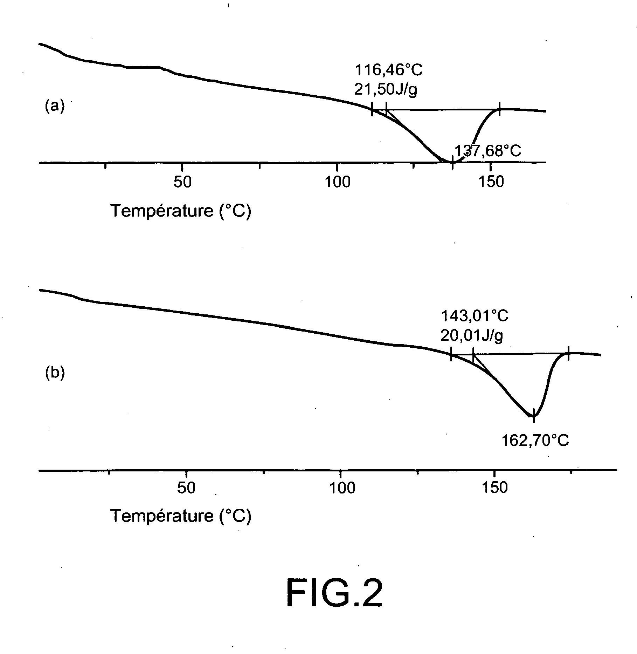

Il apparaît, en observant la figure 2, que les échantillons de polymères synthétisés avec le complexe d'yttrium 1 présentent un seul endotherme de transition, étroit, présentant ainsi la formation d'un arrangement cristallin uniforme à l'état solide, qui peut indiquer une dispersion plus étroite des stéréoséquences de la chaîne (par Pᵣ = 0,85, Tₘ = 138°C, et pour Pᵣ = 0,91, Tm = 165°C). Ce résultat est très différent des descriptions précédentes données dans la littérature, qui décrivent deux endothermes de fusion [15], ou même trois endothermes de fusion [16].

**Tableau 2**

| Polymérisation de la β-butyrolactone avec le complexe 1^{a} | | | | | | | | | |
|---|---|---|---|---|---|---|---|---|---|
| Exemple | [BBL]/[1] | [BBL] (mol.l⁻¹) | Solvant | Température (°C) | Temps (min.) | Conversion (%)^{b} | Mₙ^{c} (g.mol⁻¹) | PDI^{c} | Pᵣ^{d} |
| 1 | 200 | 2,44 | Toluène | 25 | 1 | 98 | - | - | 0,88 |
| 2 | 200 | 2,44 | Toluène | -20 | 10 | 95 | - | - | 0,91 |
| 3 | 200 | 2,44 | Chlorobenzène | 25 | 1 | 95 | - | - | - |
| 4 | 200 | 2,44 | THF | 25 | 120 | 98 | - | - | 0,83 |
| 5 | 400 | 2,44 | Toluène | 25 | 1 | 98 | 26100 | 1.06 | 0,89 |
| 6 | 400 | 1,00 | Toluène | 25 | 10 | 98 | - | - | 0,90 |
| 7 | 400 | 4,80 | Toluène | 25 | 4 | 95 | - | - | 0,87 |
| 8 | 400 | 1,00 | Toluène | -20 | - | 98 | - | - | 0,94 |
| 9 | 600 | 2,44 | Toluène | 25 | 5 | 98 | 47200 | 1.10 | - |
| 10^{e} | 800 | 2,44 | Toluène | 25 | 8 | 97 | 21900 | 1.17 | 0,90 |
| 11 | 800 | 2,44 | Toluène | 25 | 10 | 95 | - | - | - |
| 12 | 800 | 2,44 | Toluène | 50 | 3 | 96 | - | - | 0,85 |
| 13 | 800 | 2,44 | Toluène | -20 | - | 38 | - | - | 0,92 |
| 14 | 1000 | Neat | - | 25 | 5 | 95 | - | - | - |
| 15 | 2000 | 8,80 | Toluène | 25 | 18 | 87 | - | - | - |

| | | | | | | | | | |
|---|---|---|---|---|---|---|---|---|---|
| ^{a} Toutes les réactions sont réalisées avec le complexe 1. ^{b} Telle que determinée par l'intégration des résonances méthines de la BBL et du PHB en RMN du H. ^{c} *Mₙ* du H et *M_{w}*/*Mₙ* du polymère determinées par SEC-RI dans le THF à température ambiante, en utilisant des étalons polystyrène. ^{d} *P*ᵣ est la probabilité de liaisons racémiques entre les motifs monomères et est déterminée à partir de la région carbonyle du spectre du ¹³C avec trois équivalents d'iso-propanol. | | | | | | | | | |

### REFERENCES

[1] Drumright, R. E.; Gruber, P. R.; Henton, D. E. Adv. Mater. 2000, 12, 1841.
[2] Senior, P. J. and Dawes, E. A. Biochem. J. 1973, 134, 225-238.
[3] (a) Müller, H.-M.; Seebach, D. Angew. Chem. Int. Ed. Engl. 1993, 32, 477-502. (b) Sudesh, K.; Abe, H.; Doi, Y. Progr. Polym. Sci. 2000, 25, 1503-1555.
[4] (a) Doi, Y.; Kumagai, Y.; Tanahashi, N.; Mukai, K. In Biodegradable Polymers and Plastics; Vert, M., Feijen, J., Albertsson, A., Scott, G., Chiellini, E., Eds.; The Royal Society of Chemistry: Cambridge, 1992; pp 139-148. (b) Cox, M. K. In Biodegradable Polymers and Plastics; Vert, M., Feijen, J., Albertsson, A., Scott, G., Chiellini, E., Eds.; The Royal Society of Chemistry: Cambridge, 1992; pp 95-99. (c) Okada, M. Progr. Polym. Sci. 2002, 27, 87-133.
[5] Holmes, P. A. Phys. Technol. 1985, 16, 32-36.
[6] Ritter, S. K. Chemical & Engineering News 2005, 83(26), June 27.
[7] Anderson, A. J., Dawes, E. A., Microbiol. Rev. 1990, 54, 450.
[8] Okada, M. Prog. Polym. Sci. 2002, 27, 87-133.
[9] Yori, Y., Suzuki, M., Yamaguchi, A., Nishishita, T. Macromolecules, 1993, 26, 5533.
[10] (a) Billingham, N. C.; Proctor, M. G.; Smith, J. D. J. Organomet. Chem. 1988, 341, 83. (b) Moeller, M.; Kånge, R.; Hedrick, J. L. J. Polym. Sci., Part A 2000, 38, 2067. (c) Schechtman, L. A.; Kemper, J. J. PCT Int. Appl., WO 0077072, 2000.
[11] (a) Wu, B.; Lenz, R. W. Macromolecules 1998, 31, 3473. (b) Lenz, R. W.; Yang, J.; Wu, B.; Harlan, C. J.; Barron, A. R. Can. J. Microbiol. 1995, 41, 274. (c) Bloembergen, S.; Holden, D. A.; Bluhm, T. L.; Hamer, G. K.; Marchessault, R. H. Macromolecules 1989, 22, 1656.
[12] (a) Hori, Y.; Hagiwara, T. Int. J. Biol. Macromol. 1999, 25, 237. (b) Kricheldorf, H. R.; Eggerstedt, S. Macromolecules 1997, 30, 5693.
[13] Rieth, L. R., Moore, D. R., Lobkovsky, E. B., Coates, G. W. J. Am. Chem. Soc. 2002, 124, 15239.
[14] (a) Cai, C.-X.; Amgoune, A.; Lehmann, C. W.; J.-F. Carpentier Chem. Commun. 2004, 330-331. (b) Amgoune, A.; Thomas, C. M.; Balnois, E.; Grohens, Y.; Lutz, P. J.; Carpentier J.-F. Macromol. Rapid Commun. 2005, 26, 1145-1150.
[15] (a) Kricheldorf, H.R., Lee, S-R., Scharnagi, N. Macromolecules 1994, 27, 3139. (b) Gross, R.A., Zhang, Y., Konrad, G., Lenz, R. W., Macromolecules 1988, 21, 2657.
[16] Kemnitzer, J.E., McCarthy, S.P., Gross, R.A. Macromolecules 1993, 26, 1221.

## Revendications

1. Procédé de préparation d'un polymère de polyhydroxyalcanoate (PHA) par polymérisation par ouverture de cycle d'une lactone de formule (I) 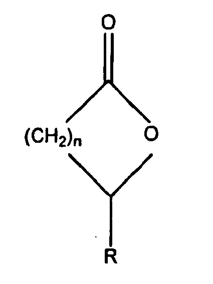 où n est un nombre entier de 1 à 4, et R représente un hydrogène ou un groupe alkyle de 1 à 4C linéaire ou ramifié, **caractérisé en ce que** la polymérisation est effectuée en présence d'un initiateur de formule (II) : 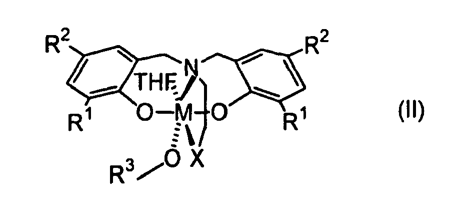 dans laquelle :
- R³ représente un groupe alkyle de 1 à 15C tel qu'un groupe méthyle, éthyle, i-propyle, n-propyle, n-butyle, i-butyle, t-butyle ; un groupe benzyle ;
- R₁ et R₂ identiques ou différents représentent chacun un groupe choisi parmi les groupes alkyle de 1 à 15C, tel que méthyle, t-butyle ; cumyle ;
(α,α-diméthylbenzyle) ; adamantyle ; trityle ; et trialkyl silyle (C₃ à C₁₅) ;
- X représente O(R₄), S(R₄) ou N(R₄)(R₅) où R₄ et R₅ représentent chacun indépendamment un groupe alkyle de 1 à 15C tel qu'un groupe méthyle, éthyle ; ou un groupe benzyle ;
- M est un métal du groupe 3 de la classification périodique des éléments tel que Y, La ou Nd.

2. Procédé selon la revendication 1, dans lequel la lactone est choisie parmi les lactones répondant aux formules suivantes : 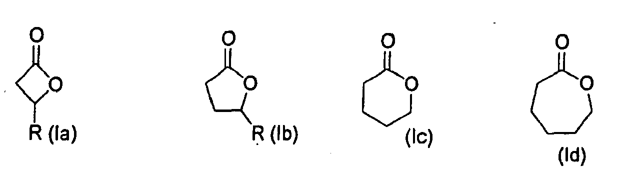 dans lesquelles R a la signification déjà donnée dans la revendication 1, et, de préférence, dans la formule (Ia), R représente H, méthyle ou, éthyle et dans la formule (Ib) R représente H, méthyle, éthyle ou propyle.

3. Procédé selon l'une quelconque des revendications précédentes, dans lequel la lactone (I) est une lactone racémique et le polyhydroxyalcanoate (PHA) préparé est un PHA syndiotactique.

4. Procédé selon la revendication 3, dans lequel la lactone est la β-butyrolactone racémique ou la γ-valérolactone racémique.

5. Procédé selon l'une quelconque des revendications 3 et 4, dans lequel le PHA préparé a un degré de syndiotacticité Pᵣ de 70 à 99%.

6. Procédé selon l'une quelconque des revendications précédentes, dans lequel l'initiateur de formule (II) est choisi parmi les composés de formules suivantes : 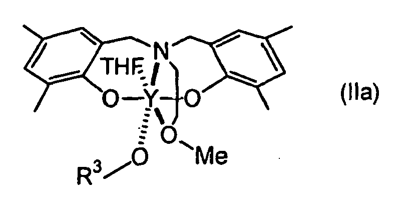 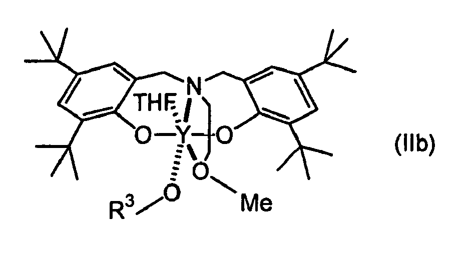 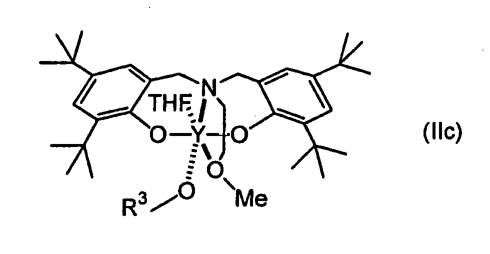 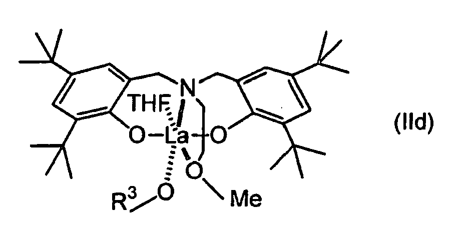 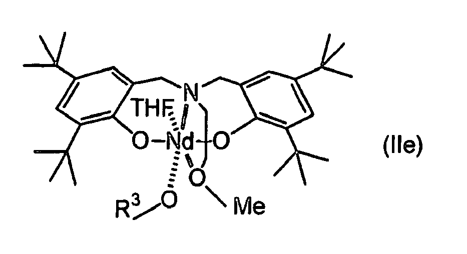 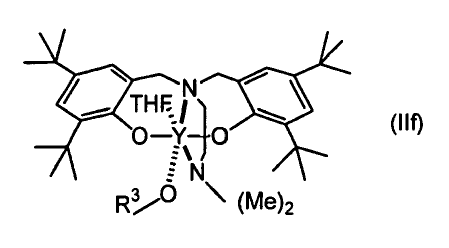 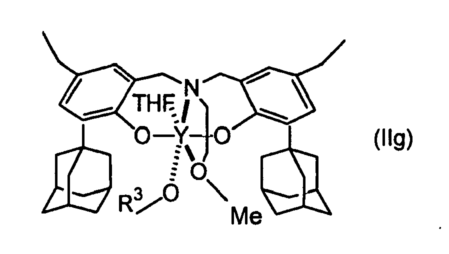 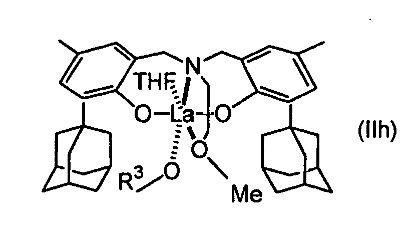 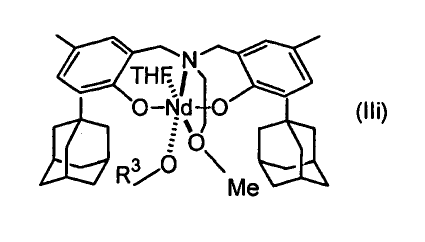 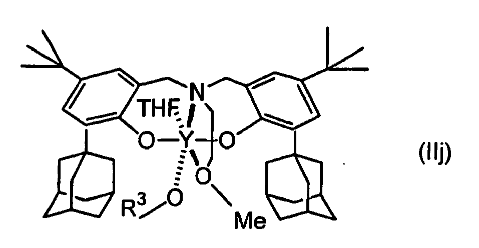 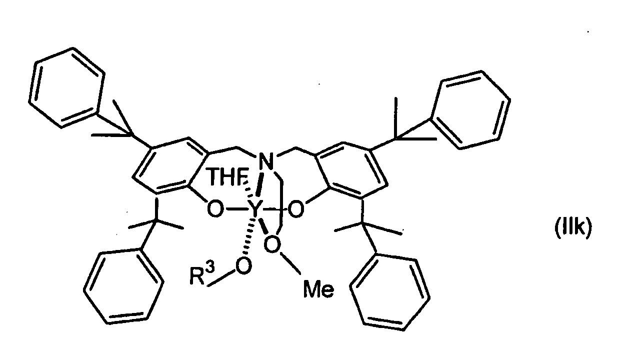 dans lesquelles R³ a la signification déjà donnée dans la revendication 1 et représente de préférence un groupe isopropyle.

7. Procédé selon l'une quelconque des revendications précédentes, dans lequel l'initiateur de formule (II) est le composé suivant :

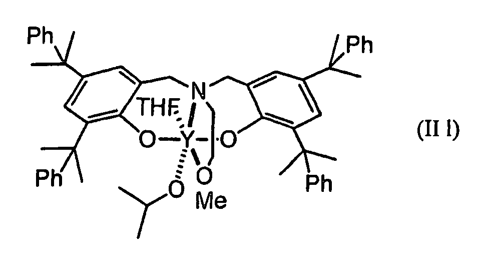

8. Procédé selon l'une quelconque des revendications précédentes, dans lequel la polymérisation est réalisée dans un solvant choisi parmi le toluène, le tétrahydrofuranne, le chlorobenzène et leurs mélanges.

9. Procédé selon l'une quelconque des revendications précédentes, dans lequel le rapport de la concentration en lactone sur celle de l'initiateur est de 50 à 5000, de préférence de 100 à 2000.

10. Procédé selon l'une quelconque des revendications précédentes, dans lequel la polymérisation est réalisée à une température de -30 à 120°C, de préférence de 0 à 60°C, de préférence encore de 15 à 30°C, par exemple de 20°C.

11. Procédé selon l'une quelconque des revendications précédentes, dans lequel la lactone est la β-butylactone racémique, le complexe est le complexe de formule (IIl) selon la revendication 5, la polymérisation est réalisée dans le toluène à une température de -30 à 110°C, de préférence de 0 à 60°C, et le rapport de la concentration en β-butyrolactone sur celle de l'initiateur est de 100 à 2000.

12. Procédé selon l'une quelconque des revendications précédentes, dans lequel le polymère préparé a une masse moléculaire moyenne en nombre de 4000 à 500 000, de préférence de 8000 ou 9000 à 170 000.

13. Procédé selon l'une quelconque des revendications précédentes, dans lequel le polymère préparé a un indice polydispersité PDI de 1,01 à 1,50, de préférence de 1,05 à 1,20.

14. Polymère de polyhydroxyalcanoate susceptible d'être obtenu par le procédé selon l'une quelconque des revendications 1 à 13, qui a une masse moléculaire moyenne en nombre de 8000 à 500 000, de préférence de 9000 à 170 000 ; un indice de polydispersité PDI de 1,01 à 1,50, de préférence de 1,05 à 1,20 ; et qui, lorsqu'il est syndiotactique, a un degré de syndiotacticité de 70 à 99%; Mn et PDi étant déterminées par SEC-Ri dans le THF à température ambiante en utilisant des étalons polystyrène.

15. Polymère selon la revendication 14, dans lequel le polyhydroxyalcanoate est un PHA syndiotactique susceptible d'être obtenu par polymérisation d'une lactone (I) racémique.

16. Polymère selon la revendication 15, dans lequel la lactone est la β-butyrolactone racémique ou la γ-valérolactone racémique.

17. Composition de matière comprenant le polymère de polyhydroxyalcanoate selon l'une quelconque des revendications 14 à 16 et d'autres ingrédients.

18. Composition selon la revendication 17, **caractérisée en ce qu'**elle est biodégradable.

19. Composition selon la revendication 18, comprenant en outre un ou plusieurs polymères biodégradables différents du polyhydroxyalcanoate.

20. Composition selon l'une quelconque des revendications 17 à 19, dans lequel le polymère de polhydroxyalcanoate est présent à raison de 0,1 à 99,9%, de préférence 1 à 99%, de préférence encore 5 à 90%, mieux 10 à 80%, mieux encore 20 à 70%, par exemple 40 à 60%, notamment 50%, ou 55% en poids de poids total de la composition.

21. Utilisation du polymère selon l'une quelconque des revendications 14 à 16 ou de la composition selon l'une quelconque des revendications 17 à 20, dans des emballages notamment alimentaires.

22. Utilisation du polymère selon l'une quelconque des revendications 14 à 16 ou de la composition selon l'une quelconque des revendications 17 à 20, dans des dispositifs et procédés biomédicaux ou pour des applications biomédicales.

23. Utilisation du polymère selon l'une quelconque des revendications 14 à 16 ou de la composition selon l'une quelconque des revendications 17 à 20, dans des fixations chirurgicales telles que des sutures, agrafes et plaques.

24. Utilisation du polymère selon l'une quelconque des revendications 14 à 16 ou de la composition selon l'une quelconque des revendications 17 à 20, pour la libération contrôlée, retardée de médicaments.

## Claims

1. Method for preparing a polyhydroxyalkanoate (PHA) polymer by ring-opening polymerization of a lactone of formula (I): 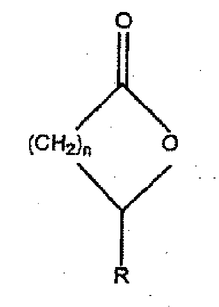 where n is an integer from 1 to 4, and R represents a hydrogen atom or a linear or branched C₁₋₄ alkyl group, **characterized in that** the polymerization is carried out in the presence of an initiator of formula (II): 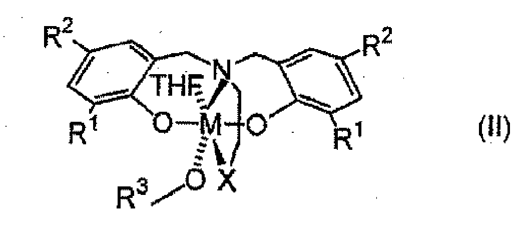 in which:
- R³ represents a C₁₋₁₅ alkyl group such as a methyl, ethyl, isopropyl, n-propyl, n-butyl, isobutyl or tert-butyl group; or a benzyl group;
- R¹ and R², being identical or different, each represent a group chosen from C₁₋₁₅ alkyl groups, such as methyl and tert-butyl groups; a cumyl group; an α,α-dimethylbenzyl group; an adamantyl group; a trityl group; and a (C₃ to C₁₅) trialkylsilyl group;
- X represents O(R₄), S(R₄) or N(R₄)(R₅) where R₄ and R₅ each independently represent a C₁₋₁₅ alkyl group such as a methyl or ethyl group; or a benzyl group;
- M is a metal from group 3 of the Periodic Table of the Elements such as Y, La or Nd.

2. Method according to Claim 1, in which the lactone is chosen from the lactones corresponding to the following formulae: 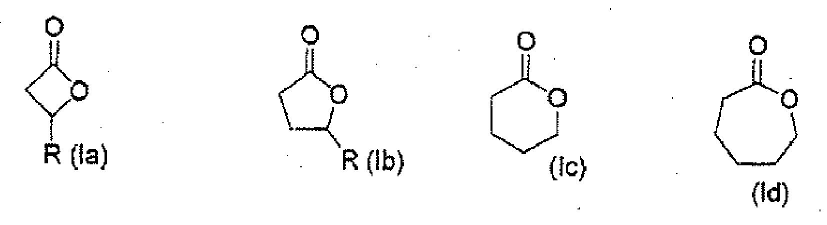 in which R has the meaning already given in Claim 1, and, preferably, in the formula (Ia), R represents H, or a methyl or ethyl group and in the formula (Ib), R represents H, or a methyl, ethyl or propyl group.

3. Method according to either one of the preceding claims, in which the lactone (I) is a racemic lactone and the polyhydroxyalkanoate (PHA) prepared is a syndiotactic PHA.

4. Method according to Claim 3, in which the lactone is racemic β-butyrolactone or racemic γ-valerolactone.

5. Method according to either one of Claims 3 and 4, in which the PHA prepared has a degree of syndiotacticity Pᵣ of 70 to 99%.

6. Method according to any one of the preceding claims, in which the initiator of formula (II) is chosen from the compounds of formulae below: 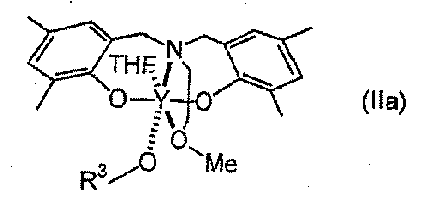 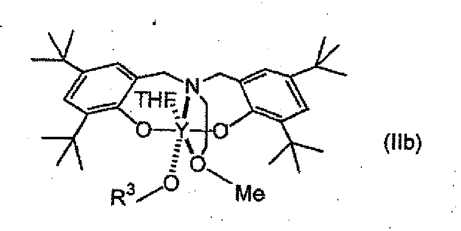 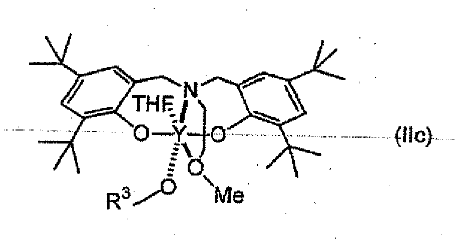 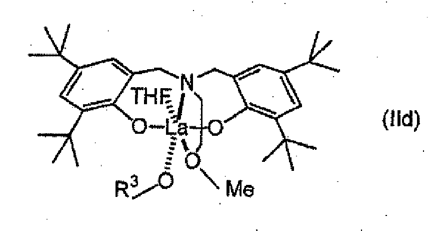 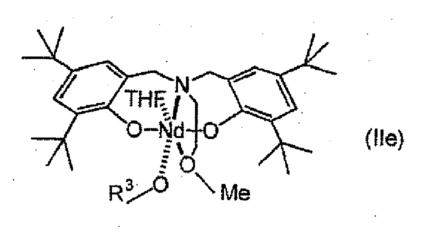 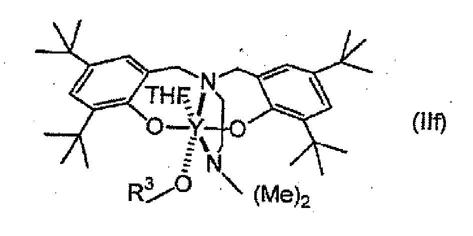 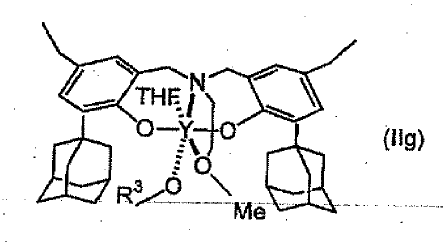 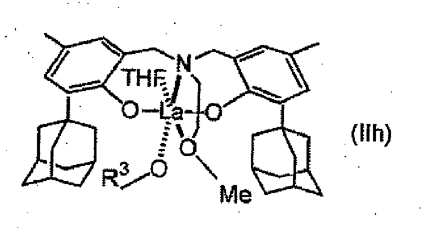 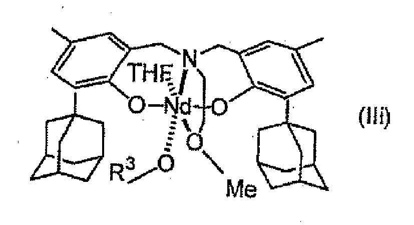 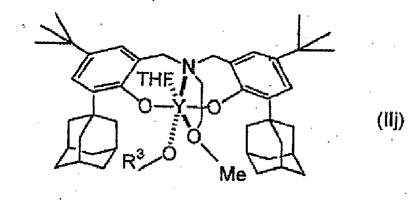 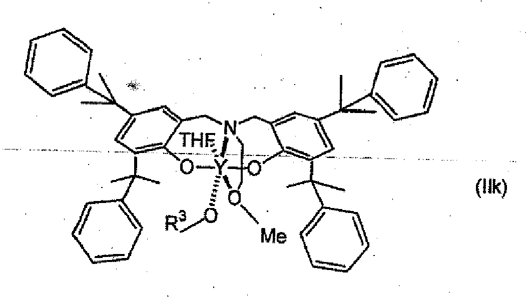 in which R³ has the meaning already given in Claim 1 and preferably represents an isopropyl group.

7. Method according to any one of the preceding claims, in which the initiator of formula (II) is the following compound:

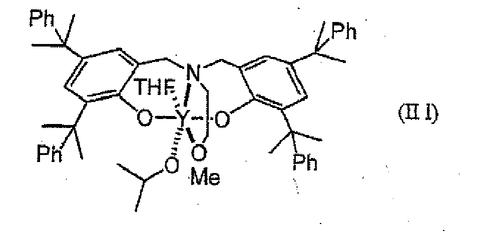

8. Method according to any one of the preceding claims, in which the polymerization is carried out in a solvent chosen from toluene, tetrahydrofuran, chlorobenzene and mixtures thereof.

9. Method according to any one of the preceding claims, in which the ratio of the lactone concentration to that of the initiator is from 50 to 5000, preferably from 100 to 2000.

10. Method according to any one of the preceding claims, in which the polymerization is carried out at a temperature from -30 to 120°C, preferably from 0 to 60°C, more preferably from 15 to 30°C, for example at 20°C.

11. Method according to any one of the preceding claims, in which the lactone is racemic β-butyrolactone, the complex is the complex of formula (III) according to Claim 7, the polymerization is carried out in toluene at a temperature from -30 to 110°C, preferably from 0 to 60°C, and the ratio of the β-butyrolactone concentration to that of the initiator is from 100 to 2000.

12. Method according to any one of the preceding claims, in which the polymer prepared has a number-average molecular weight of 4000 to 500 000, preferably from 8000 or 9000 to 170 000.

13. Method according to any one of the preceding claims, in which the polymer prepared has a polydispersity index PDI from 1.01 to 1.50, preferably from 1.05 to 1.20.

14. Polyhydroxyalkanoate polymer capable of being obtained by the method according to any one of Claims 1 to 13, which has a number-average molecular weight from 8000 to 500 000, preferably from 9000 to 170 000; a polydispersity index PDI from 1.01 to 1.50, preferably from 1.05 to 1.20; and which, when it is syndiotactic, has a degree of syndiotacticity of 70 to 99%; Mn and PDI being determined by SEC-IR in THF at ambient temperature, using polystyrene standards.

15. Polymer according to Claim 14, in which the polyhydroxyalkanoate is a syndiotactic PHA capable of being obtained by polymerization of a racemic lactone (I).

16. Polymer according to Claim 15, in which the lactone is racemic β-butyrolactone or racemic γ-valerolactone.

17. Material composition comprising the polyhydroxyalkanoate polymer according to any one of Claims 14 to 16 and other ingredients.

18. Composition according to Claim 17, **characterized in that** it is biodegradable.

19. Composition according to Claim 18, comprising, in addition, one or more biodegradable polymers different from the polyhydroxyalkanoate.

20. Composition acording to any one of Claims 17 to 19, in which the polyhydroxyalkanoate polymer is present in an amount of 0.1 to 99.9%, preferably 1 to 99%, more preferably 5 to 90%, better 10 to 80%, better still 20 to 70%, for example 40 to 60%, especially 50% or 55% by weight of the total weight of the composition.

21. Use of the polymer according to any one of Claims 14 to 16 or of the composition according to any one of Claims 17 to 20, in packaging, especially food packaging.

22. Use of the polymer according to any one of Claims 14 to 16 or of the composition according to any one of Claims 17 to 20, in biomedical devices and processes or for biomedical applications.

23. Use of the polymer according to any one of Claims 14 to 16 or of the composition according to any one of Claims 17 to 20, in surgical fasteners such as sutures, staples and templates.

24. Use of the polymer according to any one of Claims 14 to 16 or of the composition according to any one of Claims 17 to 20, for the controlled slow release of medication.

## Patentansprüche

1. Verfahren zur Herstellung eines Polyhydroxyalkanoat-Polymers (PHA-Polymers) durch ring-öffnende Polymerisation eines Lactons der Formel (I) 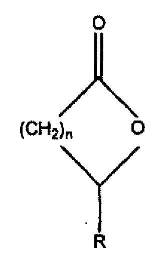 wobei n für eine ganze Zahl von 1 bis 4 steht und R für Wasserstoff oder eine lineare oder verzweigte Alkylgruppe mit 1 bis 4 C-Atomen steht, **dadurch gekennzeichnet, daß** die Polymerisation in Gegenwart eines Initiators der Formel (II): 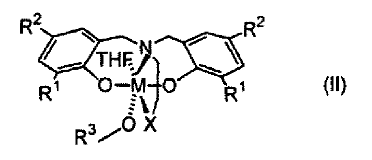 worin:
- R³ für eine Alkylgruppe mit 1 bis 15 C-Atomen, wie eine Methyl-, Ethyl-, Isopropyl-, n-Propyl-, n-Butyl-, Isobutyl- oder t-Butylgruppe; oder eine Benzylgruppe steht;
- R₁ und R₂ gleich oder verschieden sind und jeweils für eine unter Alkylgruppen mit 1 bis 15 C-Atomen, wie Methyl oder t-Butyl; Cumyl; (α,α-Dimethylbenzyl); Adamantyl; Trityl und Trialkylsilyl (mit 3 bis 15 C-Atomen) ausgewählte Gruppe stehen;
- X für O(R₄), S(R₄) oder N(R₄)(R₅) steht, wobei R₄ und R₅ jeweils unabhängig voneinander für eine Alkylgruppe mit 1 bis 15 C-Atomen wie eine Methyl- oder Ethylgruppe oder eine Benzylgruppe stehen;
- M für ein Metall der Gruppe 3 des Periodensystems der Elemente wie Y, La oder Nd steht;
durchgeführt wird.

2. Verfahren nach Anspruch 1, bei dem man das Lacton unter den Lactonen der folgenden Formeln auswählt: 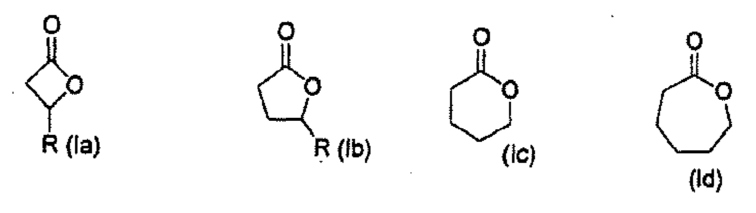 worin R die bereits in Anspruch 1 angegebene Bedeutung besitzt und R in der Formel (Ia) vorzugsweise für H, Methyl oder Ethyl steht und R in der Formel (Ib) vorzugsweise für H, Methyl, Ethyl oder Propyl steht.

3. Verfahren nach einem der vorhergehenden Ansprüche, bei dem es sich bei dem Lacton (I) um ein racemisches Lacton handelt und es sich bei dem hergestellten Polyhydroxyalkanoat (PHA) um ein syndiotaktisches PHA handelt.

4. Verfahren nach Anspruch 3, bei dem es sich bei dem Lacton um racemisches β-Butyrolacton oder racemisches γ-Valerolacton handelt.

5. Verfahren nach einem der Ansprüche 3 und 4, bei dem das hergestellte PHA einen Syndiotaktizitätsgrad Pᵣ von 70 bis 99% aufweist.

6. Verfahren nach einem der vorhergehenden Ansprüche, bei dem man den Intitiator der Formel (II) unter den Verbindungen der folgenden Formeln auswählt: 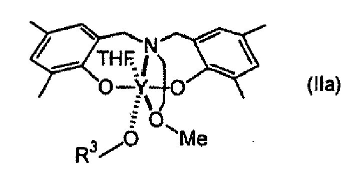 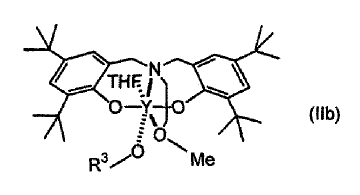 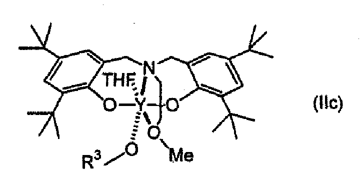 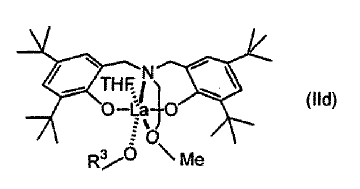 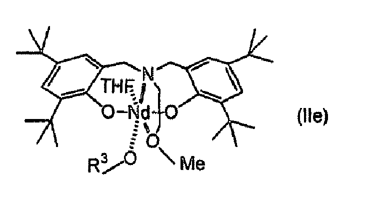 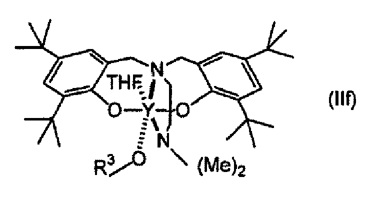 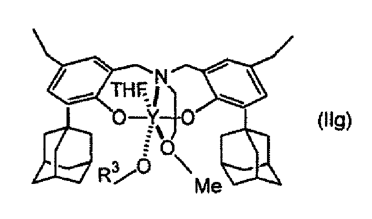 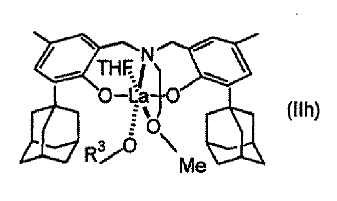 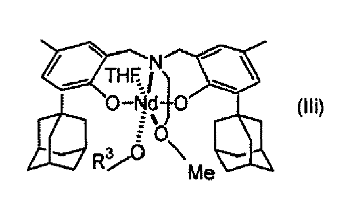 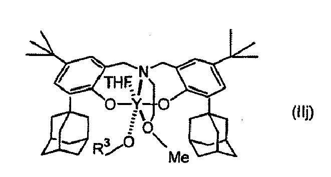 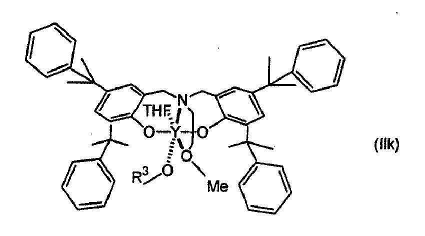 worin R³ die bereits in Anspruch 1 angegebene Bedeutung besitzt und vorzugsweise für eine Isopropylgruppe steht.

7. Verfahren nach einem der vorhergehenden Ansprüche, bei dem es sich bei dem Initiator der Formel (II) um die folgende Verbindung handelt:

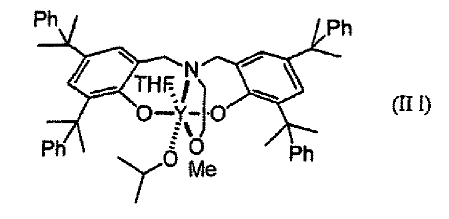

8. Verfahren nach einem der vorhergehenden Ansprüche, bei dem die Polymerisation in einem unter Toluol, Tetrahydrofuran, Chlorbenzol und Mischungen davon ausgewählten Lösungsmittel durchgeführt wird.

9. Verfahren nach einem der vorhergehenden Ansprüche, bei dem das Verhältnis von Lacton-Konzentration zu Initiator-Konzentration 50 bis 5000 und vorzugsweise 100 bis 2000 beträgt.

10. Verfahren nach einem der vorhergehenden Ansprüche, bei dem die Polymerisation bei einer Temperatur von -30 bis 120°C, vorzugsweise 0 bis 60°C und noch weiter bevorzugt 15 bis 30°C, beispielsweise 20°C, durchgeführt wird.

11. Verfahren nach einem der vorhergehenden Ansprüche, bei dem es sich bei dem Lacton um racemisches β-Butyrolacton handelt, es sich bei dem Komplex um den Komplex der Formel (III) nach Anspruch 7 handelt, die Polymerisation in Toluol bei einer Temperatur von -30 bis 110°C und vorzugsweise 0 bis 60°C durchgeführt wird und das Verhältnis von β-Butyrolacton-Konzentration zu Initiator-Konzentration 100 bis 2000 beträgt.

12. Verfahren nach einem der vorhergehenden Ansprüche, bei dem das hergestellte Polymer ein zahlenmittleres Molekulargewicht von 4000 bis 500.000 und vorzugsweise 8000 oder 9000 bis 170.000 aufweist.

13. Verfahren nach einem der vorhergehenden Ansprüche, bei dem das hergestellte Polymer einen Polydispersitätsindex PDI von 1,01 bis 1,50 und vorzugsweise 1,05 bis 1,20 aufweist.

14. Polyhydroxyalkanoat-Polymer, das nach dem Verfahren nach einem der Ansprüche 1 bis 13 erhältlich ist und ein zahlenmittleres Molekulargewicht von 8000 bis 500.000 und vorzugsweise 9000 bis 170.000, einen Polydispersitätsindex PDI von 1,01 bis 1,50 und vorzugsweise 1,05 bis 1,20 und dann, wenn es syndiotaktisch ist, einen Syndiotaktizitätsgrad von 70 bis 99% aufweist; wobei Mₙ und PDI durch SEC-IR in THF bei Umgebungstemperatur unter Verwendung von Polystyrol-Standards bestimmt werden.

15. Polymer nach Anspruch 14, bei dem es sich bei dem Polyhydroxyalkanoat um ein syndiotaktisches PHA handelt, das durch Polymerisation eines racemischen Lactons (I) erhältlich ist.

16. Polymer nach Anspruch 15, wobei es sich bei dem Lacton um racemisches β-Butyrolacton oder racemisches γ-Valerolacton handelt.

17. Stoffzusammensetzung, enthaltend das Polyhydroxyalkanoat-Polymer nach einem der Ansprüche 14 bis 16 und andere Bestandteile.

18. Zusammensetzung nach Anspruch 17, **dadurch gekennzeichnet, daß** sie biologisch abbaubar ist.

19. Zusammensetzung nach Anspruch 18, die ferner ein oder mehrere biologisch abbaubare Polymere, die von dem Polyhydroxyalkanoat verschieden sind, enthalten.

20. Zusammensetzung nach einem der Ansprüche 17 bis 19, wobei das Polyhydroxyalkanoat-Polymer in einer Menge von 0,1 bis 99,9 Gew.-%, vorzugsweise 1 bis 99 Gew.-%, noch weiter bevorzugt 5 bis 90 Gew.-%, besser 10 bis 80 Gew.-%, noch besser 20 bis 70 Gew.-%, beispielsweise 40 bis 60 Gew.-%, insbesondere 50 Gew.-% oder 55 Gew.-%, bezogen auf das Gesamtgewicht der Zusammensetzung, vorliegt.

21. Verwendung eines Polymers nach einem der Ansprüche 14 bis 16 oder der Zusammensetzung nach einem der Ansprüche 17 bis 20 in Verpackungen, insbesondere Lebensmittelverpackungen.

22. Verwendung eines Polymers nach einem der Ansprüche 14 bis 16 oder der Zusammensetzung nach einem der Ansprüche 17 bis 20 in biomedizinischen Vorrichtungen und Verfahren oder für biomedizinische Anwendungen.

23. Verwendung eines Polymers nach einem der Ansprüche 14 bis 16 oder der Zusammensetzung nach einem der Ansprüche 17 bis 20 in chirurgischen Befestigungselementen wie Nähten, Klammern und Platten.

24. Verwendung eines Polymers nach einem der Ansprüche 14 bis 16 oder der Zusammensetzung nach einem der Ansprüche 17 bis 20 zur kontrollierten, verzögerten Freisetzung von Medikamenten.
